# EUROPEAN PATENT SPECIFICATION

(11) **EP 3 972 613 B1**
(45) Date of publication and mention of the grant of the patent: **03.05.2023**
(21) Application number: 20726163.7
(22) Date of filing: 20.05.2020
(51) Int. Cl.: A61K 33/36, A61K 33/06, A61K 33/242, A61K 33/26, A61K 33/30, A61K 33/32, A61K 33/34, A61P 25/28, A61P 29/00, A61P 35/00, A61P 35/02, A61P 37/06

(54) **USE OF COPPER IONS TO POTENTIATE THE THERAPEUTIC EFFECTS OF ARSENIC**
VERWENDUNG VON KUPFERIONEN ZUR VERSTÄRKUNG DER THERAPEUTISCHEN WIRKUNGEN VON ARSEN
UTILISATION D'IONS DE CUIVRE POUR POTENTIALISER LES EFFETS DE L'ARSENIC

(30) Priority: 21.05.2019 EP 19305644; 31.05.2019 CN 201910469782
(43) Date of publication of application: 30.03.2022
(73) Proprietor: Medsenic, 67100 Strasbourg (FR)
(72) Inventor: RIEGER, François, 1203 GENEVE (CH); BATTEUX, Frédéric, 75014 PARIS (FR)
(74) Representative: Santarelli
(86) International application number: PCT/EP2020/064189
(87) International publication number: WO 2020/234414

(56) References cited:
- EP-A1- 3 459 551
- WO-A1-99/55344
- WO-A1-2019/141988
- WO-A2-2007/028154
- DE-U1- 29 721 350
- US-A- 2 280 340
- US-A1- 2004 175 432
- DATABASE CA [Online] CHEMICAL ABSTRACTS SERVICE, COLUMBUS, OHIO, US; 10 February 2000 (2000-02-10), CHI, JINGHUI: "External medicine for inhibiting cancer metastasis", XP002795510, retrieved from STN Database accession no. 2000:94575
- LIU JUANJUAN ET AL: "Alterations of antioxidant indexes and inflammatory cytokine expression aggravated hepatocellular apoptosis through mitochondrial and death receptor-dependent pathways inGallus gallusexposed to arsenic and copper", ENVIRONMENTAL SCIENCE AND POLLUTION RESEARCH INTERNATIONAL, ECOMED, LANDSBERG, DE, vol. 25, no. 16, 22 March 2018 (2018-03-22), pages 15462-15473, XP036517187, ISSN: 0944-1344, DOI: 10.1007/S11356-018-1757-0 [retrieved on 2018-03-22]
- "Dietary Reference Intakes for Vitamin A, Vitamin K, Arsenic, Boron, Chromium, Copper, Iodine, Iron, Manganese, Molybdenum, Nickel, Silicon, Vanadium, and Zinc", 2001, National Academy Press, Washington page 224,

## Description

### FIELD

The present invention pertains to the field of medicine and is based on the unexpected finding that therapeutic effects of arsenic can be potentiated by administration of Cu²⁺ ions.

### BACKGROUND

### Therapeutic use of Arsenic and possible mechanisms of action

Arsenic compounds have widely been used as traditional medicines in many parts of the world for the treatment of various diseases such as psoriasis, syphilis, or rheumatic arthritis for over two thousand years. Many different arsenic preparations have been developed and used during the long history of these agents. For example, the Fowler's solution, which contains 1% potassium arsenite (KAsO₂), was prescribed for years as a remedy for leukemias and even as a tonic.

Some arsenical compounds are notably toxic and carcinogenic, with side effects such as cirrhosis of the liver, idiopathic portal hypertension, urinary bladder cancer, and skin cancers. Recently however, several arsenic compounds have been rediscovered and formulated to treat different diseases, such as cancer.

In particular, Arsenic Trioxide (As₂O₃, also noted "ATO" in the present text) occurs to be one of the most effective novel anticancer ("antineoplastic" or "cytotoxic") agents. ATO has been approved by the US FDA for the treatment of acute promyelocytic leukemia (APL) resistant to "first line" agents, namely all-trans retinoic acid (ATRA). It has been shown that arsenic trioxide induces cancer cells to undergo apoptosis.

Arsenic Trioxide is also known or currently investigated as an agent against other diseases, such as auto-immune diseases.

Bobé et al. (Blood, 108, 13, p3967 - 3975, 2006) investigated the effects of arsenic trioxide in a mouse model of systemic lupus erythematosus. As₂O₃ significantly prolonged survival of MRLllpr mice by preventing young mice from developing the syndrome and quasi-totally reversing established disease in older animals. These authors suggested that this compound might be useful in the treatment of autoimmune diseases such as systemic lupus erythematosus.

In US 8,394,422, Chelbi-Alix and Bobé suggested that arsenic trioxide could be used for treating autoimmune and/or inflammatory diseases.

Kavian et al. (J Immunol. 2012;188(10):5142-9) reported the successful use of ATO in murine sclerodermatous GvHD.

More recently, Maier et al. (J. Immunol. 2014 Jan 15;192(2):763-70) demonstrated that arsenical compounds, including the FDA-approved anticancer therapeutic arsenic trioxide, are potent inhibitors of caspase-1 and the innate immune response (namely Interleukin 1-β) and thus may have potential for the treatment of inflammatory components of autoimmune disorders. Furthermore, Li *et al.* showed that arsenic trioxide improves Treg and T17 balance in rheumatoid arthritis patients, thus being a potential useful immune modulator (Int. Immunopharmacology, 2019, Arsenic trioxide improves Treg and Th17 balance by modulating STAT3 in treatment-naïve rheumatoid arthritis patients).

It has been previously reported that As₂O₃-induced APL cell differentiation occurs as a result of direct binding of As₂O₃ to cysteine residues of zinc fingers in the RING finger-B Box-Coiled Coil (RBCC) domain of PML-RARα fusion protein, which results in enhanced SUMOylation/Ubiquitination and then degradation of PML-RARα fusion protein, promoting cell differentiation leading to clinical remission (Zhang et al., Science. 2010;328(5975):240-3; Chen et al., Blood. 2011 Jun 16;117(24):6425-37). However, although explanatory for the beneficial action of Arsenic in this rare cancer, this was not clearly a general phenomenon that could explain the action of As in other cancerous conditions in which it was (and still is) tested for efficacy (more than 100 clinical trials are or have been conducted with arsenic compounds by 2019, as reported in clinicaltrials.gov), nor in the autoimmune diseases or graft versus host disease.

To explain the recent observations of As beneficial action on the immune system, a new mechanism of action was proposed. Indeed, it became clear that one of the major consequences of the exposure of a number of cell types to As both *in vitro* (cell lines or primary cultures, normal or diseased) and *in vivo* (animal models for autoimmune diseases) was the induction of specific cell death, generally through the stimulation of various, although generally hypothetical, proapoptotic programs, expectedly leading to changes in cell subpopulations with often decreased cytokines levels and modified cell-cell communications.

One of the main assumptions has been that some oxidative stress pathways were activated, with ROS production (sometimes interestingly observed to be not inhibited by common antioxidants (such as N-acetylcysteine-NAC)), leading to gluthathione consumption and other cell stress inducing processes leading to cell death especially in pathologically immune activated cells (see for example Bobé *et al* 2006, *supra* and Kavian *et al., supra,* suggesting plasmacytoid dendritic cell death).

To understand arsenic's antitumor mechanism further, Zhang et al. (PNAS 2015; Vol. 112, No. 49) used a human proteome microarray to identify arsenic-binding proteins. They identified 360 arsenic-binding proteins and found proteins of glycolysis to be highly enriched. They suggest that glycolysis in general and the rate-limiting enzyme hexokinase-2 of the glycolytic pathway in particular, play a key role in mediating the anticancer activity of arsenic.

Moreover, it has been reported that Arsenic targets Pin1, described as a critical "driver" and a unique drug target in cancer and other diseases. Kozono *et al.* suggest that ATO and ATRA, at clinically safe doses, cooperatively block numerous cancer-driving pathways, through direct reversible inhibition of Pin 1, so that their combination offers an attractive approach for combating breast cancer and others (Nat Commun. 2018 Aug 9;9(1):3069).

However, the detailed mechanism(s) of action of As is(are) far from being completely elucidated.

### Common adverse effects of Arsenic

As₂O₃ is a pharmacologically active compound, with highly toxic properties at high concentrations or long-term exposures.

Interestingly, treatments of Acute promyelocytic Leukemia (with FDA and EMA authorizations) and SLE (Phase 2a; terminated) or GvHDc (Phase 2, ongoing) only present reversible Adverse Effects (AE) for the intravenous (IV) dosage generally adopted (0.15 mg/kg/day over a one - two (and sometimes up to five) month periods of IV treatment).

The AEs are potential important threats to the health status of the patients, the most unwelcomed ones being a transient increase in the cardiac QT widening, possibly together with changes in blood electrolytes levels and hepatic increased release of liver enzymes. These AEs are carefully monitored, essentially reversible - but necessitate transient suspensions of the treatment for a few days - and occur in an important proportion of the patients. Typically, even when patients are recruited under strict inclusion/exclusion rules, about one third of them develop AEs.

Thus, there remains a need to decrease the amount of Arsenic administered to the patients treated with arsenic trioxide or other arsenicals to diminish the negative effects of the pharmacologically active ingredient (API; arsenic trioxide or any other arsenic compound) while keeping the same level of favorable biological activity.

However, on the one hand, the positive effects of arsenicals are dose-dependent and the doses commonly adopted are already calculated to minor the related adverse effects. On the other hand, as explained above the mechanism of action of arsenicals has been only partially determined and, by the time being, only a few observed biochemical and physiologic effects are of high value to appreciate the expected efficiency of the treatment. The most important one in the field of cancerology and immune pathologies is its ability to activate cell stress processes leading to cytokine releases and specific cell death, as well as a positive interference with specific immune mechanisms relevant to certain diseases.

Thus, there is currently a need to find drugs that would increase the positive effects of arsenic used as a medicament without exhibiting the toxicity profile of arsenic. Indeed, this would allow to decrease accordingly the amount of Arsenic salt(s) delivered for a given treatment, or to increase the efficacy of the treatment without increasing the amount of said administered arsenic salt(s).

WO2007/028154A2 discloses a composition comprising a liposome encapsulating an arsenic-containing drug and a metal for the treatment of cancer (e.g., leukemia) and autoimmune diseases, among others. The metal serves to increase the arsenic content inside the liposome and its stable loading through As-Cu aggregate formation and to allow the controlled release of arsenic ions from the aggregates under acidic pH as found in cancerous cells. No synergic effect between the arsenic and any of the metals is described.

WO2019/141988A1 discloses a pharmaceutical composition comprising arsenic trioxide, iron and an artemisinin, for the treatment of leukemia (e.g., acute myeloid leukemia). This document does not mention Copper ions.

### ROS production and related regulatory antioxidants

In this context, the inventors investigated the possibilities to increase the effect of a given dose of the API by using in parallel, drugs or molecules able to increase the cell stress.

Cell stress is mainly mediated through an increase of Radical Oxygen Species (ROS). The main cell stress oxidative pathway is well known with its cascade of steps, including:

| | | |
|---|---|---|
| i. | Generation of superoxide radical: | O₂ + 1 e⁻ → O₂^{·-} |
| ii. | Generation of hydrogen peroxide: | O₂^{·-} + O₂^{·-} + 2 H⁺ → H₂O₂ + O₂ |
| iii. | Generation of hydroxyl radical: | H₂O₂ + Fe²⁺ → ^{·}OH + Fe³⁺ + OH⁻ |

The amount of hydrogen peroxide is regulated by heme peroxidases that accelerate its dismutation and by the glutathione peroxidase (GPx) that catalyzes its reduction by glutathione (GSH).

Other ROS exist, such as peroxyl radicals RO₂^{·}, hydroperoxides RO₂H and alkoxyl radicals RO^{·}.

The H₂O₂-degrading Fenton reaction (reaction (iii) above) is typically catalyzed by free iron bivalent ions and leads to the generation of ^{·}OH. The Fenton reaction is admittedly localized at the endoplasmic reticulum or in perinuclear locations, but not in mitochondria or other cell compartments (Liu Q, et al. Proc Natl Acad Sci USA 2004;101:4302-7). Sources of H₂O₂ could be mitochondria (superoxide dismutase reaction), peroxisomes (acyl-CoA oxidase reaction) and amyloid beta of senile plaques (superoxide dismutase-like reactions). H₂O₂ that escapes the cell antioxidant machinery, such as glutathione peroxidase and catalase, might be converted nonenzymatically in a perinuclear-localized Fenton reaction and act as an RNA- or DNA-damaging agent.

### SUMMARY

The inventors investigated the possibility to increase the effect of a given dose of an arsenic compound by using in parallel, drugs or molecules able to increase the cell stress through the activation of the Fenton reaction. To this aim, they tested several elements known to increase this reaction, starting with the well-known Iron salts, as well as others, such as Zn, Mn, Mg, Cu, Au, *etc. ...*

As described in the experimental part below, they found that in a manner totally unexpected and not explainable through the mere activation of the Fenton reaction, Cu²⁺ ions specifically display strong synergic features of action with arsenic, including all searched features, *i.e.,* H₂O₂ cell production, apoptosis induction increase and physiologic effects in a basic and highly significant test for the immune system function, the Mixed Lymphocyte Reaction (MLR), developed with mouse cells of different genetic backgrounds.

The present invention thus pertains to a medicament comprising arsenic trioxide and Cu²⁺, for use in treating a disease selected from the group consisting of a neoplastic disease, an autoimmune disease, an inflammatory disease and various forms of multiple sclerosis (MS), wherein the amount of arsenic trioxide in one daily dose is between 0.01 and 0.10 mg/kg/day and wherein one daily dose comprises between 0.05 µmol/kg and 2 µmol/kg of Cu²⁺

The invention also relates to a combination of a Cu²⁺ salt and an arsenic compound, for use the treatment of various diseases including neoplastic diseases, autoimmune diseases, inflammatory diseases and various forms of multiple sclerosis (MS), wherein the arsenic compound and the Cu²⁺ salt are administered to a patient sequentially, and wherein the amount of As element in one daily dose is between 0.1 µmol/kg and 1.6 µmol/kg and the amount of Cu²⁺ in one daily dose is between 0.05 µmol/kg and 2 µmol/kg. In such a combined treatment the Cu²⁺ ions increase the therapeutic effects of arsenic.

### FIGURE LEGENDS

Figure 1: Effects of As₂O₃ at increasing concentrations on the production of H₂O₂, GSH and cell viability in HL60 cells.
Figure 2: Effects of FeSO₄ at increasing concentrations, alone or combined to As₂O₃ at 1µM, on the production of H₂O₂ and cell viability in HL60 cells.
Figure 3: Effects of HAuCl₂ at increasing concentrations, alone or combined to As₂O₃ at 1µM, on the production of H₂O₂ and cell viability in HL60 cells.
Figure 4: Effects of ZnSO₄ at increasing concentrations, alone or combined to As₂O₃ at 1µM, on the production of H₂O₂ and cell viability in HL60 cells.
Figure 5: Effects of ZnCl₂ at increasing concentrations, alone or combined to As₂O₃ at 1µM, on the production of H₂O₂ and cell viability in HL60 cells.
Figure 6: Effects of MnSO₄ at increasing concentrations, alone or combined to As₂O₃ at 1µM, on the production of H₂O₂ and cell viability in HL60 cells.
Figure 7: Effects of MnCl₂ at increasing concentrations, alone or combined to As₂O₃ at 1µM, on the production of H₂O₂ and cell viability in HL60 cells.
Figure 8: Effects of CuSO₄ at increasing concentrations, alone or combined to As₂O₃ at 1µM, on the production of H₂O₂ and cell viability in HL60 cells.
Figure 9: Effects of CuCl₂ at increasing concentrations, alone or combined to As₂O₃ at 1µM, on the production of H₂O₂ and cell viability in HL60 cells.
Figure 10: Effects of As₂O₃ (1µM) combined to CuCl₂ (1 or 4 µM) on the production of H₂O₂ and cell viability in HL60 cells.
Figure 11: Effects of As₂O₃ (1µM) combined to increasing concentrations of CuCl₂ (0.5 to 4 µM) on the production of GSH and cell viability in HL60 cells.
Figure 12: Effects of As₂O₃ on the proliferation of C57BL6 mice splenic cells in a mixed lymphocyte reaction (MLR). Ordinate: DO UptiBlue, which measures the cellular metabolic activity (and is considered as a direct growth indicator).
Figure 13: Effects of FeSO₄ at increasing concentrations, alone or combined to As₂O₃ at 1µM, on the proliferation of C57BL6 mice splenic cells in a mixed lymphocyte reaction (MLR).
Figure 14: Effects of HAuCl₂ at increasing concentrations, alone or combined to As₂O₃ at 1µM, on the proliferation of C57BL6 mice splenic cells in a mixed lymphocyte reaction (MLR).
Figure 15: Effects of ZnSO₄ and ZnCl₂ at increasing concentrations, alone or combined to As₂O₃ at 1µM, on the proliferation of C57BL6 mice splenic cells in a mixed lymphocyte reaction (MLR).
Figure 16: Effects of MnSO₄ and MnCl₂ at increasing concentrations, alone or combined to As₂O₃ at 1µM, on the proliferation of C57BL6 mice splenic cells in a mixed lymphocyte reaction (MLR).
Figure 17: Effects of CuSO₄ and CuCl₂ at increasing concentrations, alone or combined to As₂O₃ at 1µM, on the proliferation of C57BL6 mice splenic cells in a mixed lymphocyte reaction (MLR).
Figure 18: Effects of CuCl₂ at increasing concentrations, alone or combined to As₂O₃ at 1µM, on the production of H₂O₂ and cell viability in A20 cells.
Figure 19: Effects of As₂O₃ (1µM) combined to increasing concentrations of CuCl₂ (0.5 to 4 µM) on the production of GSH and cell viability in A20 cells.
Figure 20: Effects of AsI₃ at increasing concentrations on the production of H₂O₂, GSH and cell viability in HL60 cells.
Figure 21: Effects of CuCl₂ at increasing concentrations, alone or combined to AsI₃ at 1µM, on the production of H₂O₂ and cell viability in HL60 cells.
Figure 22: Effects of As₂O₃ and AsI₃ on the proliferation of C57BL6 mice splenic cells in a mixed lymphocyte reaction (MLR).
Figure 23: Effects of CuCl₂ at increasing concentrations, alone or combined to AsI₃ at 1µM, on the proliferation of C57BL6 mice splenic cells in a mixed lymphocyte reaction (MLR).

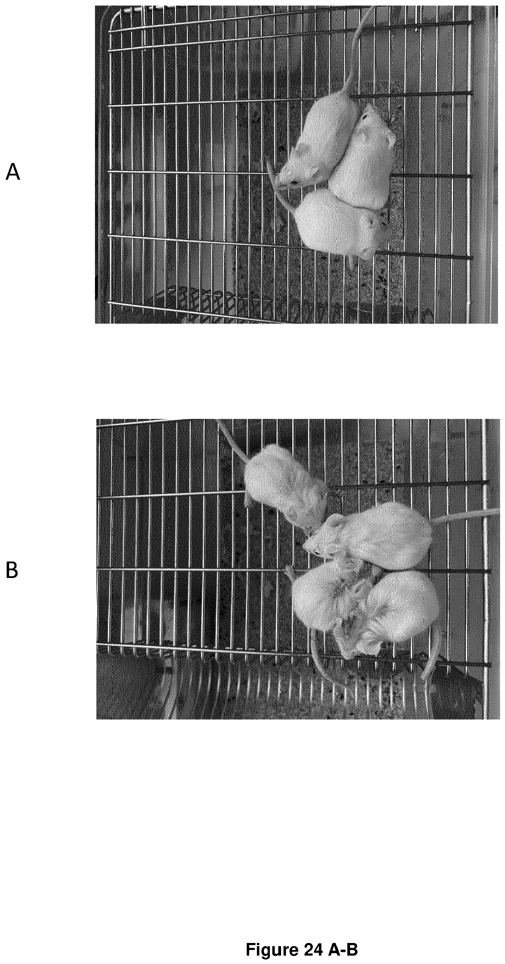

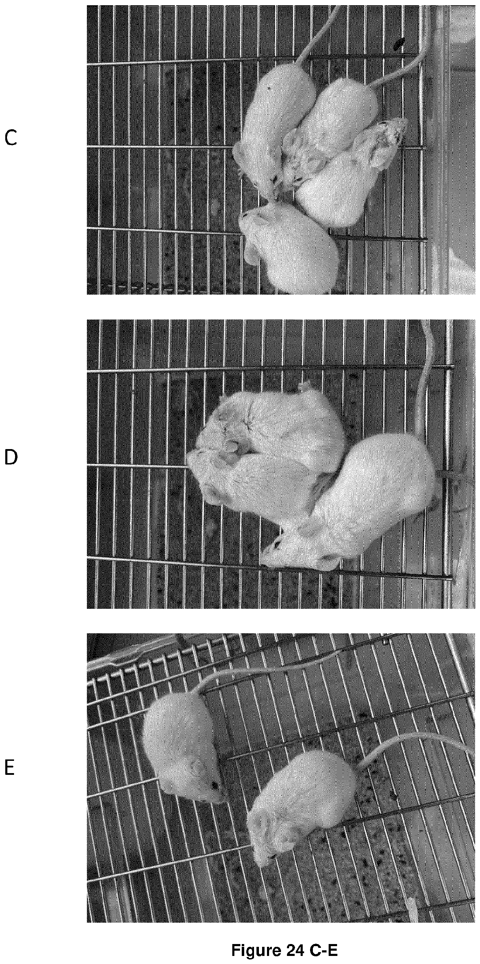

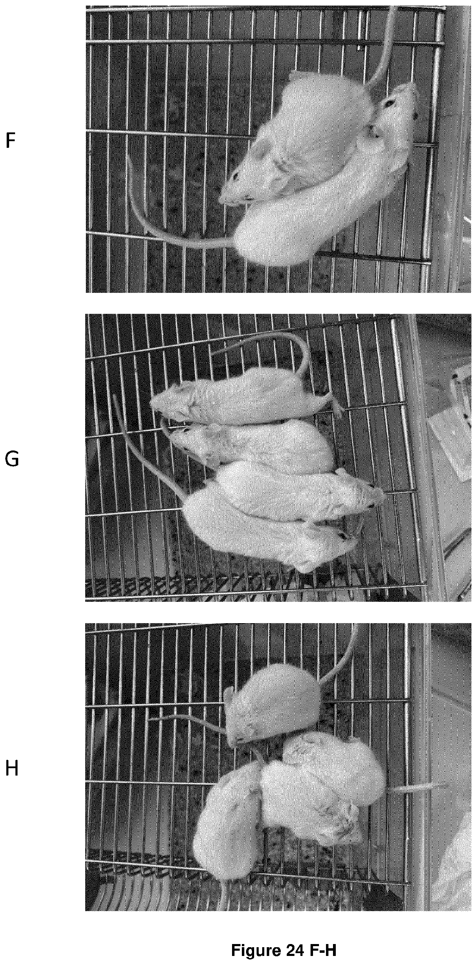

Figure 24: Effect of treatments on the onset of skin abnormalities (alopecia) in chronic GvHD. Pictures represent the development of alopecia in the different experimental groups. A: Syngeneic (8 mice). B: Allogeneic (9 mice - 7 with alopecia). C: Allogeneic + As₂O₃ 2.5 µg/g (9 mice - 3 with alopecia). D: Allogeneic + As₂O₃ 2.5 µg/g + CuCl₂ 2.5 µg/g (3 mice - none with alopecia). E: Allogeneic + CuCl₂ 2.5 µg/g (2 mice - 1 with alopecia). F: Allogeneic + As₂O₃ 2.5 µg/g + CuCl₂ 10 µg/g (2 mice - none with alopecia). G: Allogeneic + CuCl₂ 10 µg/g (5 mice - none with alopecia). H: Allogeneic + As₂O₃ 5 µg/g (5 mice - 1 with alopecia).
Figure 25: Effects of the treatments on mice weight during the evolution of GVHD. A. syngeneic and allogeneic groups treated or not with As₂O₃ (2.5 µg/g or 5 µg/g, as indicated). B. syngeneic group and allogenic groups treated or not with (As₂O₃ 2.5 µg/g and CuCl₂ 2.5 µg/g). C. syngeneic group and allogenic groups treated or not with (As₂O₃ 2,5 µg/g and CuCl₂ 10 µg/g).
Figure 26: Effects of the treatments on vasculitis (clinical scoring: ear thicknesses) during the evolution of GVHD. A. Thickness of the mice ears in the syngeneic and allogenic groups treated or not with As₂O₃ at 2.5 µg/g or 5 µg/g. B. Thickness of the mice ears in the syngeneic and allogenic groups treated or not with As₂O₃ at 2.5 µg/g and CuCl₂ at 2.5 µg/g. C. Thickness of the mice ears in the syngeneic and allogenic groups treated or not with As₂O₃ at 2.5 µg/g and CuCl₂ at 10 µg/g.
Figure 27: Effects of the treatments on the liver: transaminases levels in each mouse blood. A. ALAT in the sera of syngeneic and allogeneic mice according to the treatments. B. ASAT in the sera of syngeneic and allogeneic mice according to the treatments.

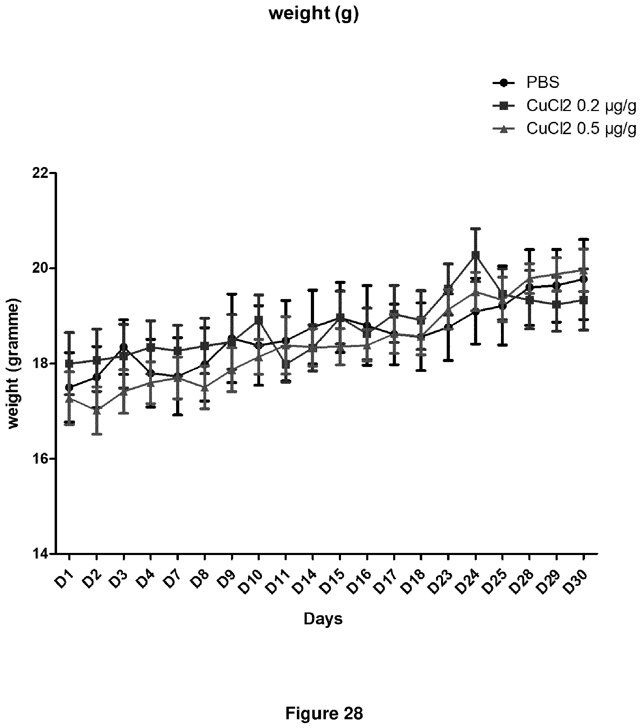

Figure 28: Effects of the treatments on mice weights during the evolution of GVHD. Mice treated with copper at 0 / 0.2 µg/g and 0.5 µg/g.

### DETAILED DESCRIPTION

As used herein, the terms "treat", "treatment" and "treating" refer to any reduction of one or more symptom(s) associated with a disease, such as, for example, a reduction of the occurrence and/or severity of symptoms in an autoimmune disease, and/or an increase in survival that results from the administration of a composition according to the invention to cancer patients. These terms are used herein to generally mean obtaining a desired pharmacologic and/or physiologic effect. The effect may be prophylactic in terms of completely or partially preventing a disease and/or may be therapeutic in terms of a partial or complete cure for a disease and/or adverse effect attributable to the disease. "Treatment" as used herein covers any treatment of a disease in a mammal, and includes: preventing the disease from occurring in a subject which may be predisposed to the disease but has not yet been diagnosed as having it; inhibiting the disease, *i.e.,* arresting its development; or relieving the disease, *i.e.,* causing regression of the disease. The therapeutic agent may be administered before, during or after the onset of disease or injury. The treatment of ongoing disease, where the treatment stabilizes or reduces the undesirable clinical symptoms of the patient, is of particular interest.

As used herein, the phrase "ameliorating at least one symptom of" refers to decreasing one or more symptoms of the disease or condition for which the subject is being treated. In particular embodiments, the disease or condition being treated is a hematological malignancy, wherein the one or more symptoms ameliorated include, but are not limited to, weakness, fatigue, shortness of breath, easy bruising and bleeding, frequent infections, enlarged lymph nodes, distended or painful abdomen (due to enlarged abdominal organs), bone or joint pain, fractures, unplanned weight loss, poor appetite, night sweats, persistent mild fever, and decreased urination (due to impaired kidney function).

As used herein, "prevent," and similar words such as "prevented," "preventing" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also include reducing the intensity, effect, symptoms and/or burden of a disease or condition prior to onset or recurrence of the disease or condition.

As used herein, the term "amount" refers to "an amount effective" or "an effective amount" of arsenic + metal ion sufficient to achieve a beneficial or desired prophylactic or therapeutic result, including clinical results. In one embodiment an effective amount refers to the amount of an arsenic-containing compound and of a metal ion sufficient to prevent, ameliorate one symptom of, or treat a disease, e.g., a hematological malignancy or an autoimmune disease contemplated herein.

A "prophylactically effective amount" refers to an amount of an arsenic-containing compound + a metal ion effective to achieve the desired prophylactic result. Typically but not necessarily, since a prophylactic dose is used in subjects prior to or at an earlier stage of disease, the prophylactically effective amount is less than the therapeutically effective amount.

A "therapeutically effective amount" of an arsenic-containing compound + a metal ion may vary according to factors such as the disease state, age, sex, and weight of the individual, and the natures of the agent (arsenic compound) and co-agent (metal ion) to elicit a desired response in the individual. A therapeutically effective amount is also one in which any toxic or detrimental effects of the agent are outweighed by the therapeutically beneficial effects. The term "therapeutically effective amount" includes an amount that is effective to "treat" a subject (e.g., a patient).

As used herein, the term "comprise" or "include" is intended to mean that the compositions and methods include the recited elements, but not excluding others. "Consist essentially of" when used to define compositions and methods, shall mean excluding other elements of any essential significance to the combination. For example, a composition consisting essentially of the elements as defined herein would not exclude other elements that do not materially affect the basic and novel characteristic(s) of the claimed invention. "Consist of" shall mean excluding more than trace amounts of other ingredients and substantial method steps recited. Embodiments defined by each of these transition terms are within the scope of this invention.

The term "about" when used before a numerical value indicates that the value may vary within reasonable range, such as ± 10%, ± 5%, and ± 1%. The expression "about x" includes the value "x."

The singular forms "a" and "the" include plural references unless the context clearly dictates otherwise. Thus, e.g., reference to "a metal ion" includes a plurality of metal ions.

Other definitions will be specified below, when necessary.

According to a first aspect, the present invention pertains to a medicament comprising arsenic trioxide and Cu²⁺, for use in treating a disease selected from the group consisting of a neoplastic disease, an autoimmune disease, an inflammatory disease and various forms of multiple sclerosis (MS), wherein the amount of arsenic trioxide in one daily dose is between 0.01 and 0.10 mg/kg/day and wherein one daily dose comprises between 0.05 µmol/kg and 2 µmol/kg of Cu²⁺.

Medicaments according to the present invention comprise arsenic trioxide, together with Cu²⁺.

According to a particular embodiment illustrated in the examples below, the Cu²⁺ ions are in the form of a salt such as copper sulfate (CuSO₄) or copper(II) chloride (CuCl₂).

The actual dosing for ATO when used in the treatment of hematologic cancers is 0.15 mg/kg/day. The results disclosed in the experimental part below demonstrate that when ATO is used in combination with Cu²⁺, its effects are potentiated. Hence, the same therapeutic efficacy should be obtained with a lower dose of ATO in the presence of Cu²⁺ as that of the currently used ATO formulation. Depending on the situation, the physician can also choose to combine with Cu²⁺ ions the same concentration of ATO as that currently approved in combination, to increase the therapeutic effects of ATO.

According to a particular embodiment of the invention, the composition according to the present invention is formulated so that one daily dose comprises between 0.01 to 0.10 mg/kg/day of arsenic trioxide (corresponding to 0.1 to 1.6 µmol/kg of arsenic atoms).

According to another particular embodiment of the invention, the composition according to the present invention is formulated so that one daily dose comprises between 0.01 to 0.05 mg/kg/day of arsenic trioxide.

According to another particular embodiment of the invention, the composition according to the present invention is formulated so that one daily dose comprises between 0.05 to 0.10 mg/kg/day of arsenic trioxide.

According to another particular embodiment of the invention, the composition according to the present invention is formulated so that one daily dose comprises between 0.05 µmol/kg and 2 µmol/kg of Cu²⁺, preferably between 0.06 µmol/kg and 2 µmol/kg of Cu²⁺, for example between 0.3 µmol/kg and 1.1 µmol/kg of Cu²⁺. According to particular embodiments, the composition according to the present invention is formulated so that one daily dose comprises about 0.05, 0.06, 0.07, 0.08, 0.09, 0.10, 0.11, 0.12, 0.15, 0.2, 0.25, 0.30, 0.35, 0.4, 0.45, 0.5, 0.6, 0.7, 0.8, 0.9, 1, 1.1, 1.2, 1.3, 1.4, 1.5, 1.6, 1.7, 1.8, 1.9, 2 µmol/kg of Cu²⁺.

Of course, the skilled person can choose any other scheme of copper administration, such as once every other day, once every three days or even once a week, to reach a dose equivalent to the daily dose described above.

Any disease which has already been successfully treated by ATO or any other arsenic compound can beneficiate from a treatment with a composition as above-described, since the combination of arsenic and

Cu²⁺ increase the beneficial effects of arsenic. In addition, one might anticipate that diseases that share pathophysiologic mechanisms with diseases and disorders already successfully treated by arsenic but that could until now not beneficiate from the effects of arsenic because they would need higher doses of active principle, difficult to reconcile with safety, could now be treated with a synergistic combination of arsenic and Cu²⁺.

The present invention thus pertains to the use of a composition as above-described in treating a disease selected amongst:
- neoplastic diseases,
- autoimmune diseases,
- inflammatory diseases and
- various forms of multiple sclerosis (MS).

The present invention also pertains to a combination of a Cu²⁺ salt (e.g., Cu₂SO₄ or CuCl₂) and an arsenic compound, for use in the treatment of a disease selected from the group consisting of a neoplastic disease, an autoimmune disease, an inflammatory disease and various forms of multiple sclerosis (MS), wherein said arsenic compound and said Cu²⁺ salt are administered to a patient sequentially, and wherein the amount of As element in one daily dose is between 0.1 umol/kq and 1.6 umol/kq and the amount of Cu²⁺ in one daily dose is between 0.05 umol/kq and 2 umol/kq.

Examples of arsenic compounds (generally salts) that can be used as active ingredients in a composition according to the invention include As₂O₃, AsI₃, As₄O₆, As₂S₂, As₂S₃, As₂S₅, As₄S₄ and mixtures thereof.

According to a particular embodiment, the composition comprising an arsenic compound is administered before the composition comprising a Cu²⁺ salt. According to another particular embodiment, the composition comprising an arsenic compound is administered after the composition comprising a Cu²⁺ salt. In both cases, the arsenic compound and the Cu²⁺ salt are administered in a time interval preferably not exceeding 12 hours.

Whatever the administration sequence, the arsenic compound and the Cu²⁺ salt are administered either via the same route or via different routes.

According to another embodiment, the present invention pertains to the use of a combination of a Cu²⁺ salt and arsenic trioxide, in the treatment of a disease selected from the group consisting of a neoplastic disease, an autoimmune disease, an inflammatory disease and various forms of multiple sclerosis (MS), wherein said arsenic trioxide and said Cu²⁺ salt are administered to a patient simultaneously, and wherein the amount of arsenic trioxide in one daily dose is between 0.01 and 0.10 mg/kg/day and the amount of Cu²⁺ in one daily dose is between 0.05 µmol/kg and 2 µmol/kg.

According to another of its aspects, the present invention pertains to the use of a Cu²⁺ salt, for potentiating/increasing the therapeutic effects of an arsenic compound as described above. Cu²⁺ ions can for example be used for potentiating the therapeutic effects of ATO or any other arsenic compound used in the treatment of a disease such as a neoplastic disease, an autoimmune disease, or more generally an inflammatory disease. By "potentiating" is herein meant that using a same amount of an arsenic compound (*e.g.*, ATO), the therapeutic effects of arsenic are significantly increased when the patient also receives Cu²⁺ ions, although the administration of Cu²⁺ ions alone is without any measurable effect. This potentiation or synergy has been demonstrated at least *in vitro,* as illustrated in examples 1 to 9 below (which show a significant increase of H₂O₂ cell production and apoptosis induction, as well as physiologic effects in a Mixed Lymphocyte Reaction (MLR), which is a highly significant assay for the immune system function). The results shown in example 10 illustrate this synergy *in vivo,* in an animal model.

Depending on the context (nature of the arsenic compound, disease concerned, stage of this disease and general parameters of the patient), the clinician will adapt the dosing and scheme of administration of arsenic compound and Cu⁺²

According to a particular embodiment of the invention, the patient is administered a daily dose that comprises between 0.01 to 010 mg/kg/day of arsenic trioxide, during 1 to 80 days.

According to another particular embodiment, the patient is administered a daily dose that comprises between 0.01 to 0.05 mg/kg/day of arsenic trioxide, during 1 to 80 days, with the possibility to reiterate the treatment if needed for reaching a satisfactory level of clinical efficacy.

According to another particular embodiment, the patient is administered a daily dose that comprises between 0.05 to 0.10 mg/kg/day of arsenic trioxide, during 1 to 80 days.

According to another particular non-claimed aspect of the disclosure, the patient is administered a daily dose that comprises between 0.10 to 0.15 mg/kg/day of arsenic trioxide, during 1 to 80 days.

According to another particular embodiment of the invention, the patient is administered a daily dose that comprises between 0.05 µmol/kg and 2 µmol/kg of Cu²⁺, preferably between 0.06 µmol/kg and 2 µmol/kg of Cu²⁺, for example between 0.3 µmol/kg and 1.1 µmol/kg of Cu²⁺, as long as he/she receives the arsenic compound. According to particular embodiments, the patient receives about 0.05, 0.06, 0.07, 0.08, 0.09, 0.10, 0.11, 0.12, 0.15, 0.2, 0.25, 0,30, 0.35, 0.4, 0.45, 0.5, 0.6, 0.7, 0.8, 0.9, 1, 1.1, 1.2, 1.3, 1.4, 1.5, 1.6, 1.7, 1.8, 1.9, 2 µmol/kg of Cu²⁺, each day, as long as he/she receives the arsenic compound.

The Cu²⁺ ions can be, for example, in the form of copper sulfate or copper(II) chloride.

As already mentioned, the arsenic compound and the Cu²⁺ salt are administered to the patient sequentially, through the same or a different route of administration. The Cu²⁺ salt can be administered to the patient every day, every other day, two or three times a week, or even weekly.

According to a particular embodiment, the arsenic compound is administered intravenously.

According to another particular embodiment, the arsenic compound is administered orally.

According to another particular embodiment, the arsenic compound is administered topically.

According to another particular embodiment, the arsenic compound is administered as an aerosol.

### Neoplastic diseases

The term "neoplastic disease" is used herein to refer to a pathologic proliferation of cells in tissue of a subject, as compared with normal proliferation in the same type of tissue. Neoplasms include benign tumors and malignant tumors (e.g., leukemias or colon tumors or prostate cancer) that are either invasive or noninvasive. Malignant neoplasms are distinguished from benign neoplasms in that the former show a greater degree of anaplasia, or loss of differentiation and orientation of cells, and have the properties of invasion and metastasis

Cancers that can be treated according to the present invention include solid and non solid cancers. According to a particular embodiment, the cancer is a hematological malignancy. Example of hematological malignancies that can be treated according to the invention include acute myeloid leukemia; acute nonlymphocytic leukemia; myeloblastic leukemia, promyelocytic leukemia; chronic myelomonocytic leukemia; monocytic leukemia; erythroleukemia; acute neutrophilic leukemia; myelodysplastic syndrome; acute promyelocytic leukemia; chronic lymphocytic leukemia; chronic myeloid leukemia; hairy cell leukemia; myeloproliferative neoplasms; Hodgkin's lymphoma; non-Hodgkin's lymphoma; myeloma; giant cell myeloma; indolent myeloma; localized myeloma; multiple myeloma; plasma cell myeloma; sclerosing myeloma; solitary myeloma; smoldering multiple myeloma; nonsecretary myeloma; osteosclerotic myeloma; plasma cell leukemia; solitary plasmacytoma; and extramedullary plasmacytoma.

In another embodiment, said hematological malignancy is acute promyelocytic leukemia (APL). In one embodiment, said APL is newly diagnosed APL. In another embodiment, said APL is relapsed or refractory APL.

Other cancers that can be treated according to the present invention include, without limitation, carcinomas (e.g., squamous-cell carcinomas, adenocarcinomas, hepatocellular carcinomas, and renal cell carcinomas), particularly those of the bladder, bowel, breast, cervix, colon, esophagus, head, kidney, liver, lung, neck, ovary, pancreas, prostate, and stomach; benign and malignant melanomas; myeloproliferative diseases; sarcomas, particularly Ewing's sarcoma, hemangiosarcoma, Kaposi's sarcoma, liposarcoma, myosarcomas, peripheral neuroepithelioma, and synovial sarcoma; tumors of the central nervous system (e.g., gliomas, astrocytomas, oligodendrogliomas, ependymomas, gliobastomas, neuroblastomas, ganglioneuromas, gangliogliomas, medulloblastomas, pineal cell tumors, meningiomas, meningeal sarcomas, neurofibromas, and Schwannomas); germ-line tumors (e.g., bowel cancer, breast cancer, prostate cancer, cervical cancer, uterine cancer, lung cancer, ovarian cancer, testicular cancer, thyroid cancer, astrocytoma, esophageal cancer, pancreatic cancer, stomach cancer, liver cancer, colon cancer, and melanoma); mixed types of neoplasias, particularly carcinosarcoma and Hodgkin's disease; and tumors of mixed origin, such as Wilms' tumor and teratocarcinomas.

### Autoimmune and inflammatory diseases

Autoimmune diseases are diseases of the immune system characterized by the production of antibodies (called autoantibodies) which react with antigens (called autoantigens) originating from the tissues of the patient or diseases which are characterized by an activation of immune cells (for example cytotoxic cells) with or without the production of antibodies.

Inflammatory diseases include a vast array of disorders and conditions that are characterized by inflammatory processes, either secondary or primary or even unique (prevalent over other immune mechanisms).

Autoimmune diseases and inflammatory diseases that can be treated with a combination of an arsenic compound and a metal ion such as Cu²⁺ according to the invention include, without limitation, systemic lupus erythematosus (SLE); acute disseminated lupus erythematosus; Bechet's disease; juvenile arthritis; Fiessinger-Leroy-Reiter syndrome; gout; osteoarthrosis; polymyositis; myocarditis; autoimmune rheumatoid arthritis (RA); systemic vasculitis; insulin-dependent diabetes mellitus (IDDM; type I diabetes, inflammatory bowel disease (IBD); celiac disease, autoimmune thyroid disease; Sjögren's syndrome, autoimmune gastritis, ulcerative colitis; Crohn's disease; autoimmune hepatitis, primary biliary cirrhosis; primary sclerosing cholangitis; cutaneous autoimmune diseases; autoimmune dilated cardiomyopathy, multiple sclerosis (MS) and other demyelinating diseases; myasthenia gravis (MG); vasculitis (e.g., Takayasu's arteritis and Wegener's granulomatosis); aplastic anemia, any disease associated with a nontumoral lymphoproliferation; B-lymphocyte lymphoma; Simmonds' syndrome; subacute thyroiditis and Hashimoto's disease; Addison's disease; autoimmune diseases of the muscle, autoimmune neuromuscular disorders, such as ankylosing spondylitis, multiple sclerosis, its varied forms including pediatric syndromes and acute disseminated encephalitis; immune mediated neuropathies; autoimmune diseases of testis, autoimmune ovarian disease, autoimmune uveitis, Graves' disease, psoriasis, ankylosing spondylitis, Addison disease, Hashimoto thyroiditis, idiopathic thrombocytopenic purpura, autoimmune lung disease such as Wegener's disease and Churg-Strauss syndrome; immunologic lung diseases such as asthma, infiltrative lung disease, hypersensitivity lung disease and sarcoidosis; dermatomyositis including scleroderma and polymyositis; and vitiligo.

The products of the present invention can also be used for treating graft-versus-host disease (GvHD) either as a preventive as well as a curative treatment.

### Multiple sclerosis

The beneficial effects of arsenic compounds, especially arsenic trioxide, has already been reported for multiple sclerosis (US 2018/325944). Thus, multiple sclerosis and related syndromes can be treated according to the invention with a combination of an arsenic compound and a metal ion such as Cu²⁺.

Particular diseases that can be treated according to the present invention are human diseases or their animal counterparts which are already successfully treated with ATO, such as acute promyelocytic leukemia, systemic lupus erythematosus (SLE), chronic graft versus host disease (GvHD), multiple sclerosis (MS), Sjögren syndrome, rheumatoid arthritis, Crohn's disease, myelocytic leukemia, chronic myeloid leukemia, chronic lymphocytic leukemia, malignant glioma, myelodysplastic syndrome, multiple myeloma and liver cancer. As explained above, the present invention provides a means to potentiate the effects of arsenic when used in the treatment of these diseases, thus enabling to use lower daily doses of ATO or to obtain better therapeutic results with the same dosage as currently used.

Other characteristics of the invention will also become apparent in the course of the description which follows of the biological assays which have been performed in the framework of the invention and which provide it with the required experimental support, without limiting its scope.

### EXAMPLES

Absent any indication to the contrary, the experimental results shown in Examples 1 to 9 have been obtained using the following materials and methods.

### a- Cells, Animals and Chemicals

*HL60 Cell culture:* the HL-60 cell line is a Human caucasian promyelocytic leukaemia cell line (ATCC n°CCL-240). This line was maintained throughout the experiment, retaining 2×10⁵ cells per ml by Flask (Falcon 250mL 75 cm², Reference: 353135) in a RPMI 1640 + GlutaMax culture medium Sigma-Aldrich (Saint-Quentin Fallavier, France) containing 10% Foetal Bovine Serum (Gibco, USA), 1% penicillin-streptomycin (Gibco, USA), 1% ciprofloxacine and 1% fungizone (Gibco,USA).

*A20 Cell culture:* the A20 cell line is a mouse cell line (ATCC n°TIB-208). This line was maintained throughout the experiment, retaining 2×10⁵ cells per ml by Flask (Falcon 250mL 75 cm², Reference: 353135) in a RPMI 1640 + GlutaMax culture medium Sigma-Aldrich (Saint-Quentin Fallavier, France) containing 10% Foetal Bovine Serum (Gibco, USA), 1% penicillin-streptomycin (Gibco, USA), 1% ciprofloxacine, 1% fungizone (Gibco, USA) and 1% 2-mercaptoethanol (Gibco, USA).

*Animals:* eight week-old female BALB/c and female C57BI6 mice were purchased from Janvier Labs (Le Genest-Saint-Isle, France) and maintained with food and water ad libitum. They were given humane care, according to national guidelines. The spleen cells of each type mouse were used on the culture mixed of lymphocytes (model MLR).

### Chemicals :

- The first five divalent cations tested were purchased from Sigma-Aldrich (Saint-Quentin Fallavier, France): CuSO₄, FeSO₄, MnSO₄, ZnSO₄ as well as HAuCl₂ (Laboratory grades).
- The three others divalent cations tested have been purchased from ChemCon (Freiburg, Germany): CuCl₂, MnCl₂ and ZnCl₂ (GMP grades).
- Arsenic trioxide (Arscimed, quality clinical batch, stock solution at 1mg/ml) come from MEDSENIC (Strasbourg, France).

### b- Statistical analysis

All of the quantitative data were analyzed with GraphPad Prism5, using one-way ANOVA and a post-hoc (Tukey). A p value < 0.05 was considered statistically significant.

### c- Effects of As₂O₃ with or without divalent cations on H₂O₂ production and GSH production in HL-60 cultured cells (Note that the divalent cations other than Cu2+ do not form part of the present invention)

### Conditions of culture and treatment

Cells (5×10⁵ per well) were seeded in 96-wells round bottom plates (Falcon, Corning, reference: 353077) and incubated for 48 hours with different experimental conditions in an incubator at 37°C 5% CO₂. Two plates were prepared in parallel to measure the H₂O₂ production and HL-60 cell viability, respectively.

### Measurement of H₂O₂ production

After 48 hours, the supernatant was removed and 50 µL per well of 50 µg/mL 2', 7'-dichlorodihydrofluorescein diacetate (Sigma-Aldrich Saint-Quentin Fallavier, France) in PBS was added on cells in same conditions of treatment (medium, or As₂O₃ with or without cation).

The H₂O₂ production was assessed by spectrofluorimetry using a fusion spectrofluorimeter (Packard). Fluorescence intensity was recorded immediately (T0 hour) and after 6 hours of incubation (T6 hours). Fluorescence excitation/emission maxima were for 2', 7'-dichlorodihydrofluorescein diacetate 485/530 nm.

### Measurement of GSH production

After 48 hours, the supernatant was removed and 50 µL per well of 50 µg/mL monochlorobimane (Sigma-Aldrich Saint-Quentin Fallavier, France) in PBS was added on cells in same conditions of treatment (medium, or As₂O₃ with or without cation).

The GSH production was assessed by spectrofluorimetry using a fusion spectrofluorimeter (Packard). Fluorescence intensity was recorded immediately (T0 hour) and after 6 hours of incubation (T6 hours). Fluorescence excitation/emission maxima were for monochlorobimane, 380/461 nm.

### HL-60 cell viability

The medium was removed and cells were stained with 0.5% crystal violet and 30% ethanol in PBS for 30 minutes at room temperature. After two washes in PBS, the stained cells were suspended in methanol, and absorbance was measured at 550nm by a fusion spectrofluorimeter (as in Ngô et al., Reactive Oxygen Species Controls Endometriosis Progression; The American journal of pathology; 2009, 175(1):225-34).

### Calculation of H₂O₂ or GSH produced during 6 hours :

The H₂O₂ or GSH production by the cells were calculated in each condition of treatment as follows:

### Fluorescence intensity [arbitrary units] at T6h - fluorescence intensity [arbitrary units] at T0 DO produced by viable cells with membrane integrity

### Conditions of treatments and related experimentations (2 tests)

- *H₂O₂ or GSH production and cell survival:* range of As₂O₃: 0.5; 1; 5 and 10 µM
- *H₂O₂ production:* comparison of 5 bivalent cations at 4 concentrations with or whithout AS₂O₃ (1 µM):
   ∘ Sigma:
      CuSO₄: 0.5; 1; 2 and 4 µM
      FeSO₄: 0.5; 1; 2 and 4 µM
      ZnSO₄: 6; 12.5; 25 and 50 µM
      HAuCl₂: 0.125; 0.25; 0.5; 1 µM
      MnSO₄: 0.125; 0.25; 0.5; 1 µM
   ∘ Chemcon:
      CuCl₂: 0.5; 1; 2 and 4 µM
      ZnCl₂: 6; 12.5; 25 and 50 µM
      MnCl₂:125; 0.25; 0.5; 1 µM

### Additional results

- *H₂O₂ or GSH production and cell survival:* range of As₂O₃ concentrations at 0.5; 1; 5 and 10 µM with CuCl₂ at 1 and 4 µM (1 test)
- *GSH production and cell survival:* As₂O₃ (1 µM) with CuCl₂ at 0.5, 1, 2 and 4 µM (2 tests)

### d- Effects of As₂O₃ with or without divalent cations on H₂O₂ production and GSH production in A20 cultured cells

### Conditions of culture and treatment

Cells (1×10⁵ per well) were seeded in 96-wells round bottom plates (Falcon, Corning, reference: 353077) and incubated for 48 hours with different experimental conditions in an incubator at 37°C 5% CO₂. Two plates were prepared in parallel to measure respectively the H₂O₂ production and A20 cell viability.

*H₂O₂ production, GSH production and cell viability* were measured using the same protocols as above-described for H60 cells.

*Conditions of treatments and related experimentations: 1 test H₂O₂* - *1 test GSH*
- *H₂O₂ or GSH production and cell survival:* As₂O₃ (1 µM) with CuCl₂ at 0.5; 1; 2 and 4 µM.

### e- As₂O₃ with or without divalent cations on the proliferation of C57BI6 CD4 T cells in vitro (Cell proliferation in mixed lymphocyte cultures).

The model is based on the mixed culture of lymphocytes derived from the suspensions of spleen cells from a female C57BI6 mouse and a female BALB/c mouse irradiated at 30 Gy. The spleen cells were mechanically separated and the erythrocytes were eliminated by hypotonic lysis of (ACK - NH₄Cl 0.15M + KHCO₃ 1mM + Na₂EDTA 0.1mM).

The UptiBlue Viable Cell Counting assay was used to measure quantitatively the *in vitro* cell proliferation. C57BI6 cells (responding cells) were seeded with irradiated BALB/c cells (stimulating cells) at 6×10⁵ per well per line in 96-wells around bottom plates (Falcon, Corning, reference: 353077). The mixed cells culture was incubated for 48 hours at different experimental conditions in an incubator at 37°C 5% CO₂.

After 48h, 10 µl of UptiBlue Viable Cell Counting assay (Interchim, reference: UP669413) was added directly in culture medium and the C57 cell proliferation was measured using a spectrofluorimeter (Fusion microplate reader fluorometer, Packard) after 24 hours of incubation with UptiBlue at 37°C with 5% CO₂.

***Conditions of treatments:*** 2 tests were performed (except with ZnCl₂ and MnCl₂)
∘ Sigma:
   CuSO₄: 0.5; 1; 2 and 4 µM
   FeSO₄: 0.5; 1; 2 and 4 µM
   ZnSO₄: 6; 12.5; 25 and 50 µM
   HAuCl₂: 0.125; 0.25; 0.5; 1 µM
   MnSO₄: 0.125; 0.25; 0.5; 1 µM
∘ Chemcon:
   CuCl₂: 0.5; 1; 2 and 4 µM
   ZnCl₂: 6; 12.5 ; 25 and 50 µM
   MnCl₂:125; 0.25; 0.5; 1 µM

### Example 1: Effects of As₂O₃ on H₂O₂ and GSH production and cell survival of HL60 cells

The HL60 cell line is a human leukemia cell line. When a patient has leukemia, he undergoes chemotherapy to eliminate leukemia cells and then receives a bone marrow transplant from a healthy patient. In some cases, hematopoietic stem cell transplantation induces chronic Graft-versus Host Disease (GvHD). First-line therapy for chronic GVHD is based on immunosuppressive agents (corticosteroids with or without cyclosporine) achieving satisfactory response in around 30% of patients. A Phase II study is currently conducted by Medsenic to evaluate if the addition of arsenic trioxide to standard therapy is effective in controlling chronic GvHD and to reduce the duration of corticosteroid therapy. In this study, arsenic trioxide is administrated to patients at 0.15mg/kg/day. One aspect of this study is to see if arsenic trioxide is able to act on possibly surviving leukemic cells resistant to chemotherapy, in addition to its effect on the autoimmune features of chronic GvHD, through a process involving the induction of cellular stress on the cells.

Cells are able to respond to cellular stress by inducing production of reactive oxygen species. In this study, investigators observed the effect of As compounds/As₂O₃-induced cell stress in the presence of divalent cations on the production of hydrogen peroxide (H₂O₂), Glutathione production (GSH), and cell viability.

In order to induce cell stress, the HL60 cells (5×10⁵ cells/well/strain) were seeded in 96-well plates (Falcon, Corning, reference: 353077) and incubated for 48 hours in complete medium with or without arsenic trioxide at several concentrations: 0.1; 0.5; 1; 5 and 10µM (As₂O₃) in an incubator at 37°C 5% CO₂.

### Effects of As₂O₃:

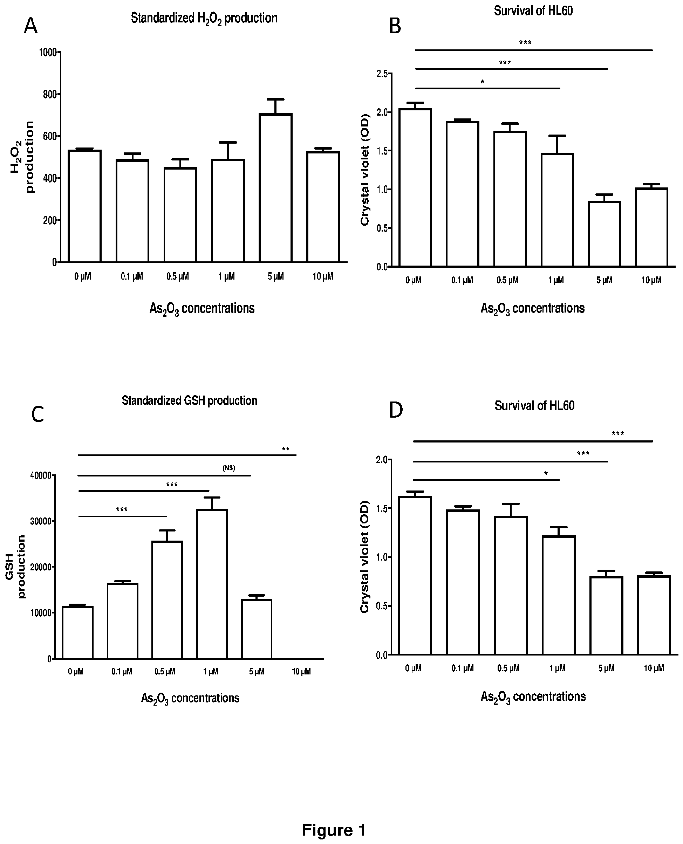

Figure 1 shows the effect of As₂O₃ at increasing concentrations on the production of H₂O₂, GSH and cell viability (4 independent tests).

The production of H₂O₂ in the presence of As₂O₃ is not significant, but it increases at 5 µM (Figure 1A).

GSH production increases significantly in the presence of As₂O₃ at 0.5, 1 and 5 µM, and disappears at 10 µM (Figure 1C).

HL-60 cell viability decreases significantly in the presence of As₂O₃ at 1, 5 and 10 µM (p<0.05, p<0.001 and P<0.001 respectively; Figures 1B and 1D).

Cell death at 5 and 10µM explains the decrease of H₂O₂ and GSH production at these As₂O₃ concentrations.

### Conclusion

The dose of 1 µM of As₂O₃ was selected to test the effect of combinations of As₂O₃ and metal ions on H₂O₂ production and cell survival of HL60 cells.

At this concentration, the biological effect of H₂O₂ production is still compensated by the production of GSH and the cell viability is preserved. Thus, the As₂O₃ effect can be modulated by a metal ion.

### Example 2: Effects of combinations of As₂O₃ and metal ions on H₂O₂ production and cell survival of HL60 cells

In order to induce cell stress, the HL60 cells (5×10⁵ cells/well/strain) were seeded in 96-well plates (Falcon, Corning, reference: 353077) and incubated for 48 hours in complete medium with or without arsenic trioxide 1 µM (As₂O₃) with or without different cations or with culture medium alone in an incubator at 37°C 5% CO₂. The results presented come from 2 independent experiments (except MnCl₂ and ZnCl₂), and the cations have different quality levels: *laboratory grade* CuSO₄ (0.5; 1; 2 and 4 µM), FeSO₄ (0.5; 1; 2 and 4 µM), ZnSO₄ (6; 12.5; 25 and 50µM), HAuCl₂ (0.125; 0.25; 0.5; 1µM), MnSO₄ (0.125; 0.25; 0.5; 1µM) and *GMP grade* CuCl₂ (0.5; 1; 2 and 4µM), ZnCl₂ (6; 12.5; 25 and 50µM), MnCl₂ (125; 0.25; 0.5; 1µM).

### Effects of combinations of As₂O₃ and metal ions:

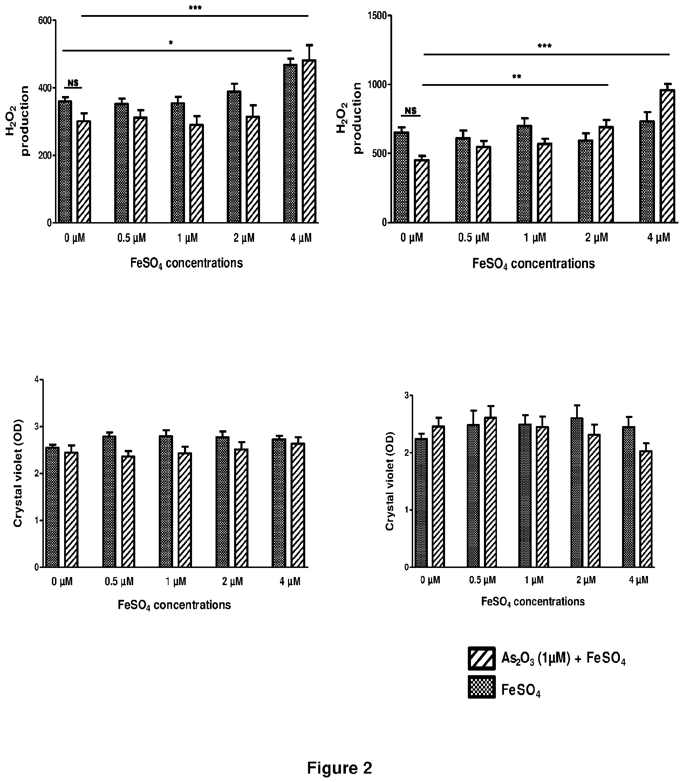

The results of these experiments on HL60 cell for H₂O₂ production and cell viability with FeSO₄, HAuCl₂, ZnSO₄, ZnCl₂, MnSO₄, MnCl₂, CuSO₄ and CuCl₂ are illustrated in Figures 2 to 9, respectively.

The statistically significant differences are represented on the figures as follows:
- control cells vs. cells treated by As₂O₃
- control cells vs. cells treated by cations only
- cells treated by As₂O₃ vs. cells treated by As₂O₃ + cations.

FeSO₄ alone has a significant effect on H₂O₂ production only at 4 µM (test 1), or with As₂O₃ (1µM) at 4 µM (test 1, p<0,001) and at 2 and 4 µM (test 2, p<0,01 and 0.001, respectively (Figure 2, upper panels). FeSO₄ alone and with As₂O₃ (1µM) has no significant effect on HL60 viability (Figure 2, lower panels).

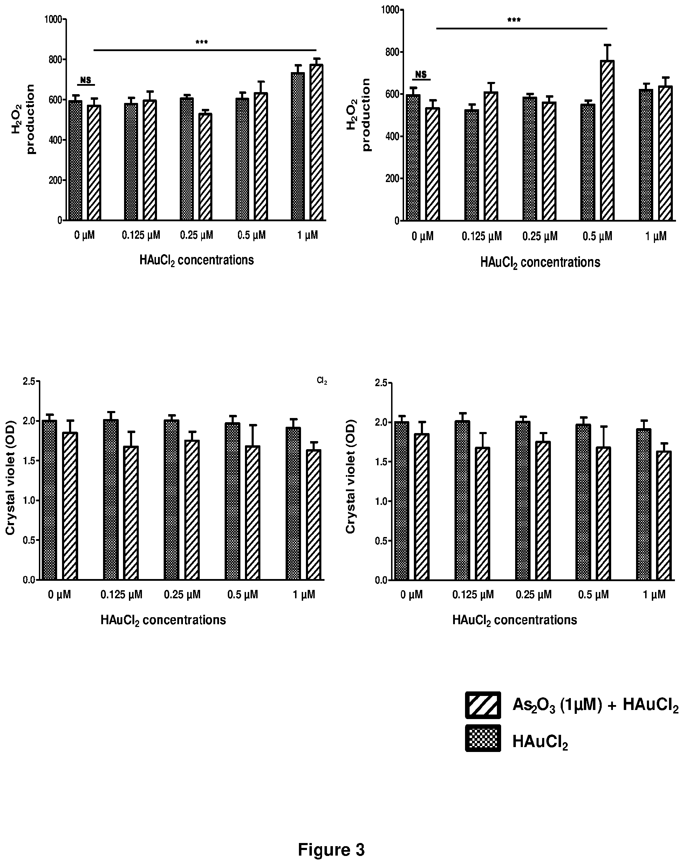

HAuCl₂ alone has no significant effect on H₂O₂ production; this effect is limited at 1 µM with As₂O₃ (p<0.01, test 1) and at 0.5 µM with As₂O₃ (p<0.01, test 2; Figure 3, upper panels). HAuCl₂ alone and with As₂O₃ (1µM) has no significant effect on HL60 viability (Figure 3, lower panels).

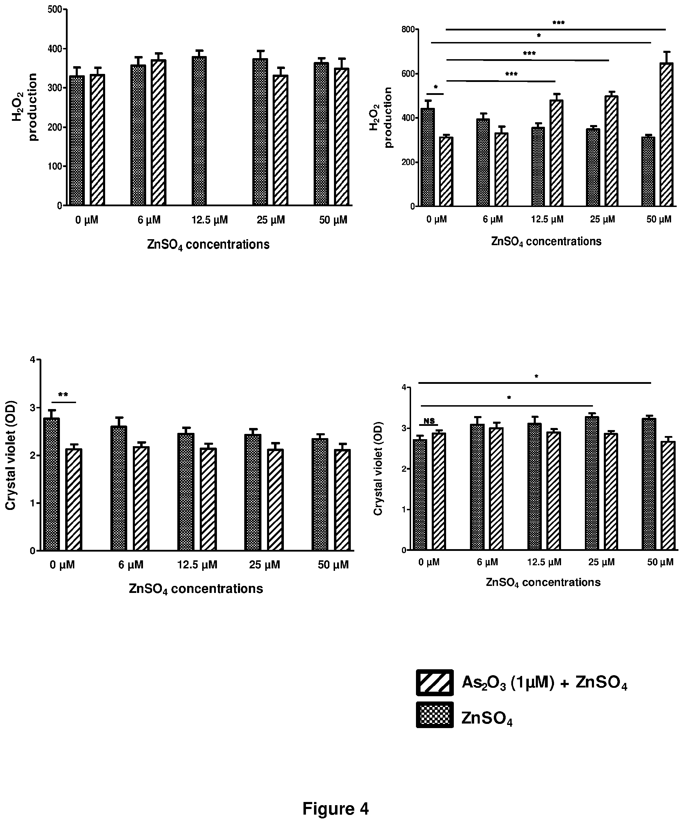

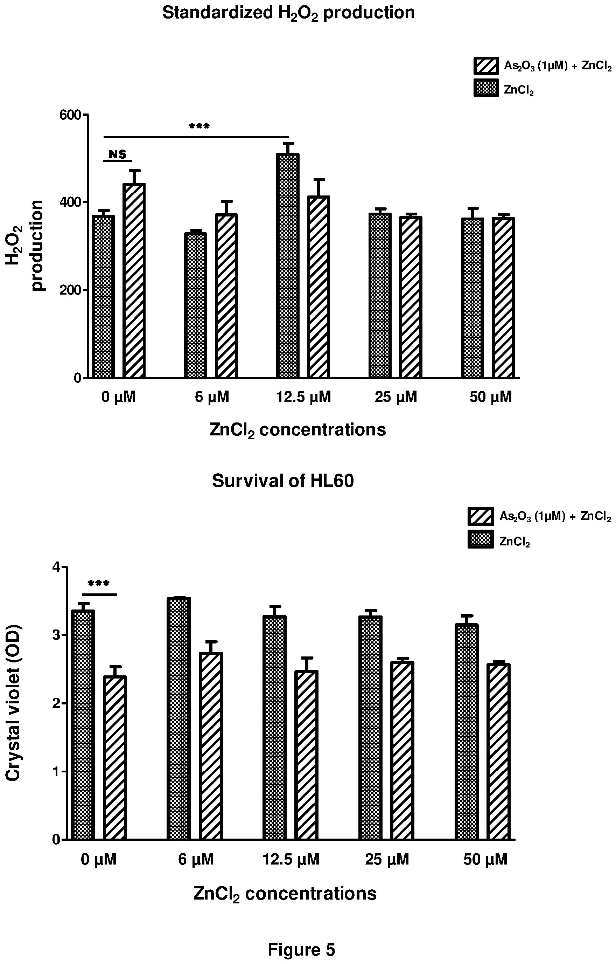

ZnSO₄ and ZnCl₂ alone have no reproducible effect on H₂O₂ production (Figures 4 and 5, upper panels). With As₂O₃ (1 µM), ZnSO₄ significantly increases H₂O₂ production at 12.5; 25 and 50 µM (p<0.001, only test 2; Figure 4, right-hand upper panel). With As₂O₃, ZnSO₄ and ZnCl₂ have no significant and reproducible effect on cell viability (Figures 4 and 5, lower panels).

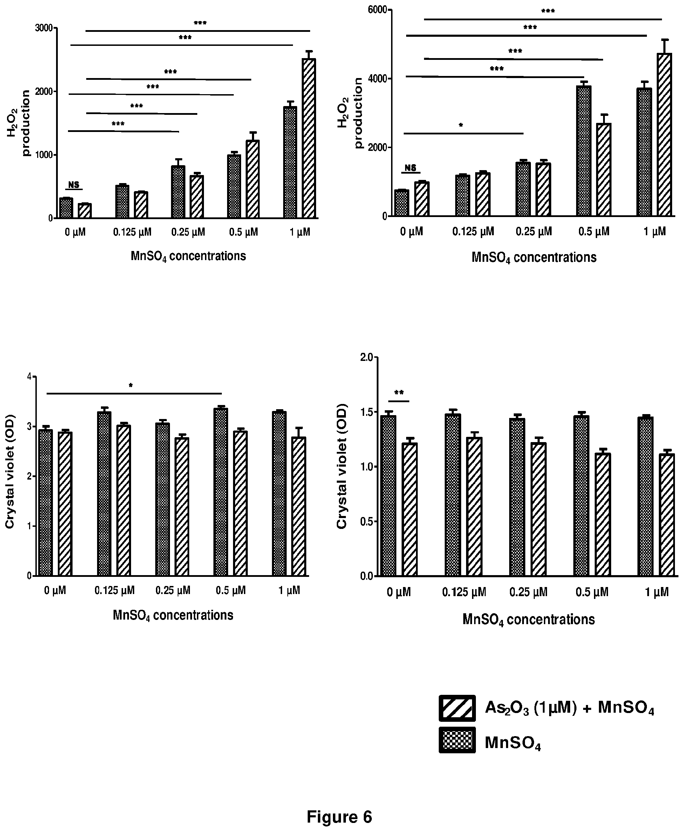

MnSO₄ has a significant dose-dependent effect on H₂O₂ production at 0.25, 0.5 and 1 µM (tests 1 and 2, Figure 6, upper panels) and additional effect with As₂O₃ at 0.5 and 1 µM (p<0.001; tests 1 & 2; Figure 6, upper panels) and even with As₂O, at 0.25 (p<0.001; test 2; Figure 6, left-hand upper panel). MnSO₄, with or without As₂O₃, have no real effect on cell viability (Figure 6, lower panels).

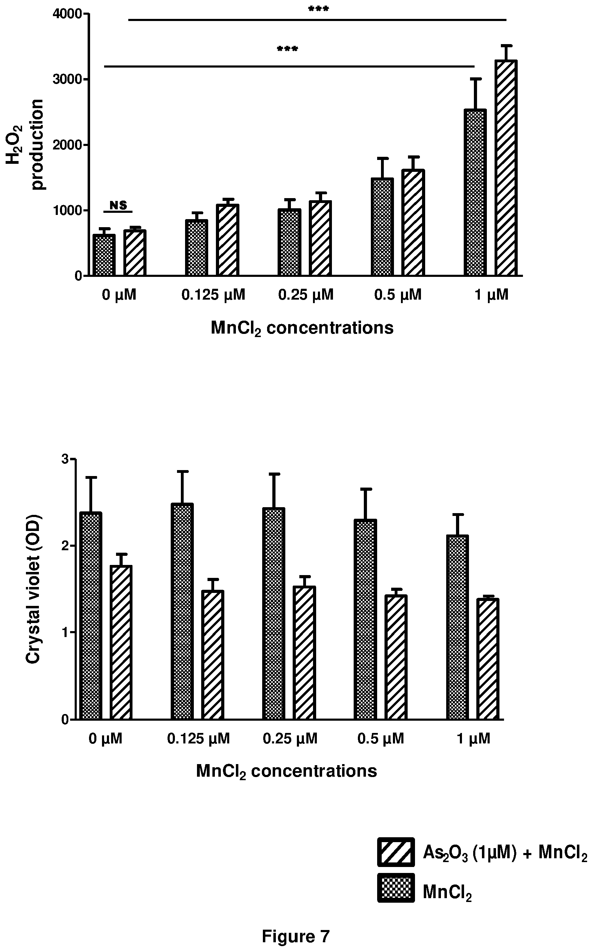

MnCl₂, with or without As₂O₃ has a dose-dependent effect on H₂O₂ production, but the increases are only significant at 1 µM (p<0.001, test 1, Figure 7, upper panel). MnSO₄, with or without As₂O₃, has no real effect on cell viability (Figure 7, lower panel).

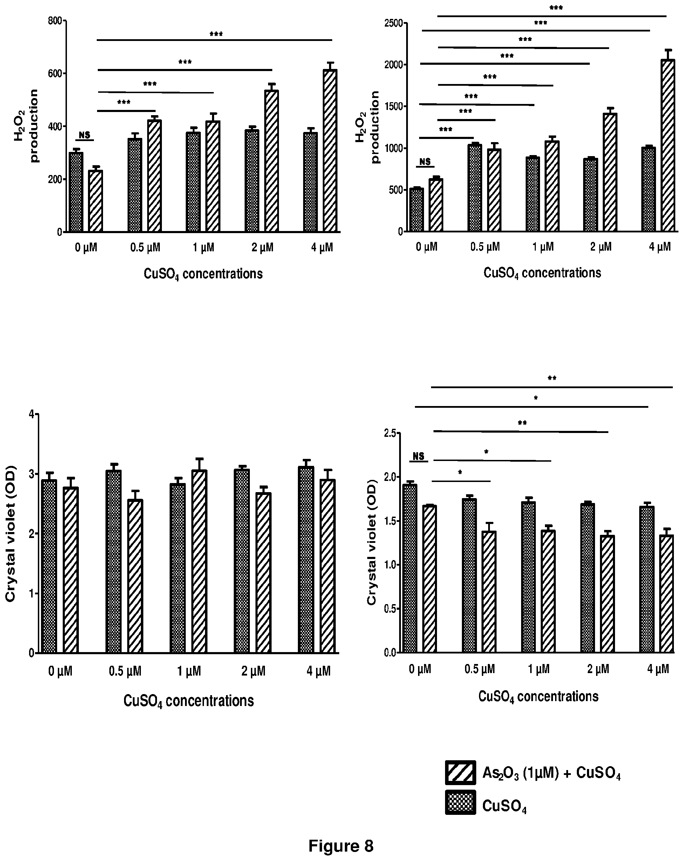

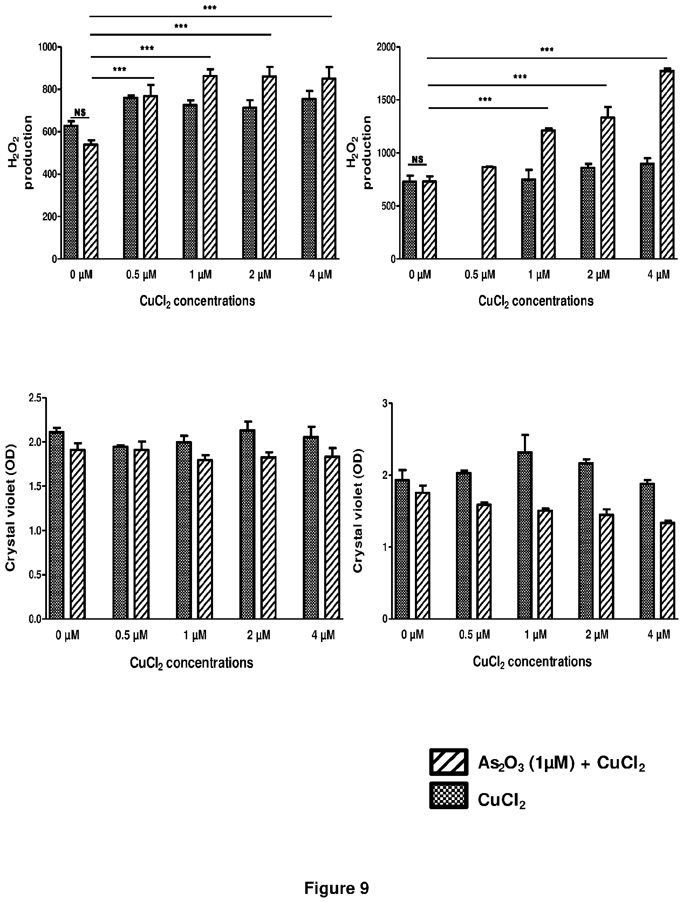

CuSO₄ and CuCl₂ alone have no significant effect on H₂O₂ production (Figures 8 and 9, upper panels). The apparent effect of CuSO₄ on H₂O₂ production, in test 2, can be explained by an abnormally low control value. CuSO₄ and CuCl₂ both increase the effect of As₂O₃, at all concentrations tested (p<0.001, Figures 8 and 9, upper panels). CuSO₄ and CuCl₂, with or without As₂O₃, seem to have no real effect on cell viability (Figures 8 and 9, lower panels).

Table 1 summarizes the results obtained on H₂O₂ production by HL60.

**Table 1**

| **Cations** | **H₂O₂ Production: cations vs control** | **H₂O₂ Production: As₂O₃ + cations vs As₂O₃** | **Cell survival: cations vs control** | **Cell survival: As₂O₃ + cations vs As₂O₃** |
|---|---|---|---|---|
| **FeSO₄ 2 tests** | 4 µM (test 1) | 4 µM (test 1) | Not significant (tests 1 and 2) | Not significant (tests 1 and 2) |
| | Not significant (test 2) | 2 and 4 µM (test 2) | | |
| **HAuCl₂ 2 tests** | Not significant (tests 1 and 2) | 1 µM (test 1) | Not significant (tests 1 and 2) | Not significant (tests 1 and 2) |
| | | 0.5 µM (test 2) | | |
| **ZnSO₄ 2 tests** | Not significant (test 1) | Not significant (test 1) | Not significant (test 1) | Not significant (tests 1 and 2) |
| | 50 µM (test 2) | 12.5, 25 and 50 µM (test 2) | 25 and 50 µM (test 2) | |
| **ZnCl₂ 1 test** | 12.5 µM | Not significant | Not significant | Not significant |
| **MnSO₄ 2 tests** | 0.25, 0.5, 1 µM (tests 1 and 2) | 0.25, 0.5 and 1 µM (test 1) | 0.5 µM (test 1) Not significant (test 2) | Not significant (tests 1 and 2) |
| | | 0.5 and 1 µM (test 2) | | |
| **MnCl₂ 1 test** | 1 µM | 1 µM | Not significant | Not significant |
| **CuSO₄ 2 tests** | Not significant (test 1) | 0.5, 1, 2 and 4 µM (tests 1 and 2) | Not significant (test 1) | Not significant (test 1) |
| | 0.5, 1, 2 and 4 µM (test 2) | | 4 µM (test 2) | 0.5, 1, 2 and 4 µM (test 2) |
| **CuCl₂ 2 tests** | Not significant (tests 1 and 2) | 0.5, 1, 2 and 4 µM (test 1) | Not significant (tests 1 and 2) | Not significant (tests 1 and 2) |
| | | 1, 2 and 4 µM (test 2) | | |

| | | | | |
|---|---|---|---|---|
| *Note:* several tests have been performed to check the effect of a sequential deposit of As₂O₃ followed by metal ions (or the opposite: metal ions + As₂O₃) or a mix of the two actives ingredients. No significant difference was found between the series of tests (data not shown). | | | | |

### Conclusion

Copper ions have no effect on HL60 viability, alone or in the presence of As₂O₃ but significantly increase the effect of As₂O₃ on H₂O₂ production, in a dose-dependent manner. CuCl₂ was chosen for further analysis because it has (and is immediately available in industrial quantities with) a GMP quality grade.

### Example 3: Effects of combination of As₂O₃ and CuCl₂ on H₂O₂ and GSH production and cell survival of HL60 cells.

In order to induce cell stress, the HL60 cells (5×10⁵ cells/well/strain) were seeded in 96-well plates and incubated for 48 hours in complete medium with or without arsenic trioxide at several concentrations (0.1; 0.5; 1; 5 and 10µM) and with and without CuCl₂ at 1 and 4 µM in an incubator at 37°C 5% CO₂.

### Effects of the combination of As₂O₃ and CuCl₂ :

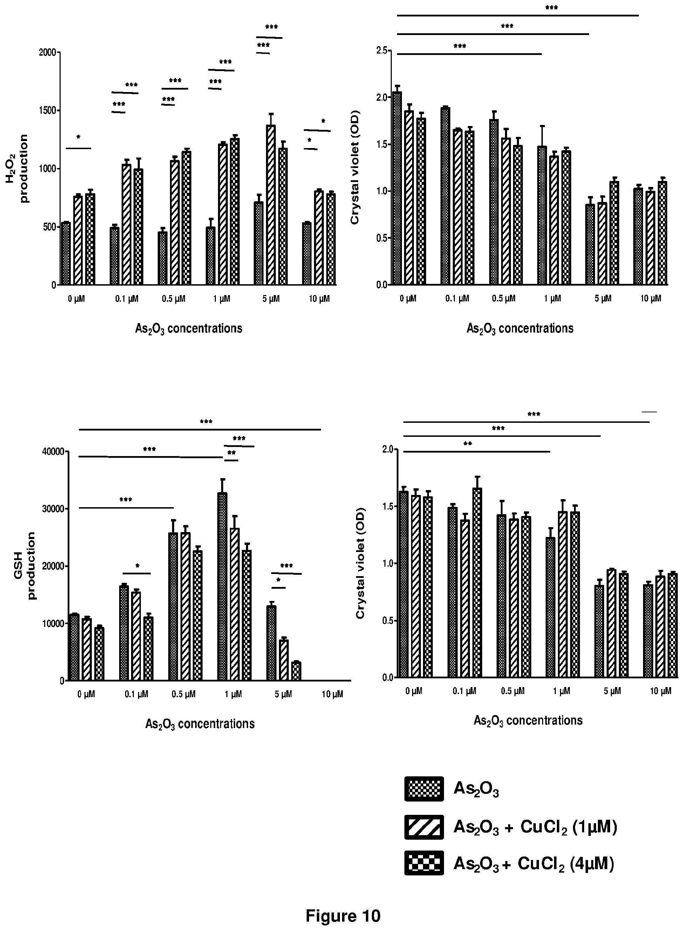

CuCl₂ at 1 and 4 µM significantly increases the H₂O₂ production induced by As₂O₃ at all concentrations (Figure 10, left-upper panel).

CuCl₂ at 1 and 4 µM reduce the GSH production induced by As₂O₃, mainly at 1 and 5 µM of As₂O₃ (Figure 10, left-lower panel).

CuCl₂ at 1 and 4 µM has no additional nor protective effect on HL60 survival in the presence of As₂O₃ (Figure 10, right-hand panels).

### Conclusion

CuCl₂, tested at 1 and 4 µM with a large range of As₂O₃ concentrations, shows no additional nor protective effect on HL60 survival but has an additional effect on H₂O₂ production and a negative effect on GSH production.

### Example 4: Effects of combination of As₂O₃ and CuCl₂ on GSH production and cell survival of HL60 cells.

In order to induce cell stress, the HL60 cells (5×10⁵ cells/well/strain) were seeded in 96-well plates and incubated for 48 hours in complete medium with or without arsenic trioxide at 1 µM and with and without CuCl₂ at 0.5, 1, 2 and 4 µM in an incubator at 37°C 5% CO₂.

### Effects of the combination of As₂O₃ (1 µM) and CuCl₂ (0.5, 1, 2 and 4 µM)

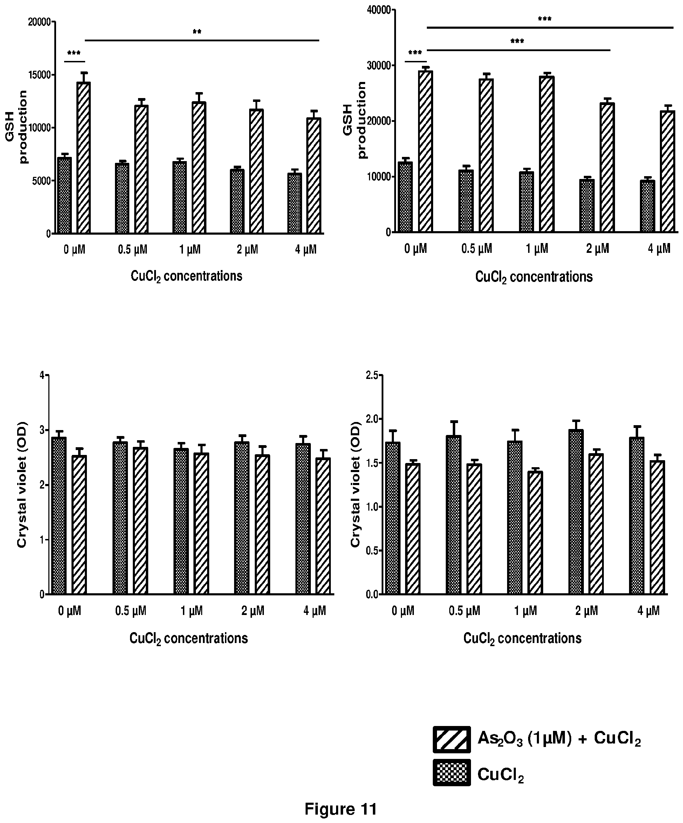

As₂O₃ at 1 µM significantly increases GSH production. In test 1, CuCl₂ reduces the increase of GSH induced by As₂O₃, but this effect was only observed at higher concentrations (Figure 11, upper panels).

CuCl₂ alone, at any of the 4 tested concentrations, has no significant effect on GSH production (Figure 11, upper panels ).

CuCl₂, at any of the 4 tested concentrations, alone or with As₂O₃, has no significant effect on cell viability (Figure 11, lower panels ).

Table 2 displays the significant results on GSH production.

**Table 2**

| | **GSH Production: cations vs control** | **GSH Production: As₂O₃ + cations vs As₂O₃** | **Cell survival: cations vs control** | **Cell survival: As₂O₃ + cations vs As₂O₃** |
|---|---|---|---|---|
| **CuCl₂** : **(2 tests)** | Not significant (tests 1 and 2) | 4 µM (test 1) | Not significant (tests 1 and 2) | Not significant (tests 1 and 2) |
| | | 2 and 4 µM (test 2) | | |

### Conclusion

These results confirm the strong potentiating action of copper on As₂O₃-induced oxidative stress on HL60 cells.

### Example 5: Effects of combinations of As₂O₃ and metal ions on the proliferation of C57BL6 mice splenic cells in a mixed lymphocyte reaction (MLR)

Mixed lymphocyte reaction (MLR) is an *ex vivo* cellular immune assay that occurs between two allogeneic lymphocyte populations (same species but genetically distinct). The two populations of lymphocytes are incubated together, and the reaction that occurs is measured.

Here, MLR is used as a model of autoimmune reaction which corresponds to an *in vitro* proliferation of splenic cells from C57BL6 mice exposed to and stimulated by irradiated splenic cells from BalbC mice. This *in vitro* technique mimics the lymphocyte reaction that happens *in vivo* in graft versus host disease (GvHD) following allogenic hemapoietic stem cells transplant.

*In vitro* cell proliferation assays were performed on spleen cells from C57BL6 mice co-cultured with previously *in vitro* irradiated (30 Gray) BALB/c spleen cells.

For proliferation assays, the cells (6×10⁵ cells/well/strain) were inoculated in 96-well plates (Falcon, Corning, reference: 353077) and incubated for 48 hours in complete medium with or without arsenic trioxide 1 µM (As₂O₃), with or without different cations or with culture medium alone in an incubator at 37°C 5% CO₂. The results presented come from 2 independent experiments, and the cations have different quality levels, as described above in the section regarding materials and methods.

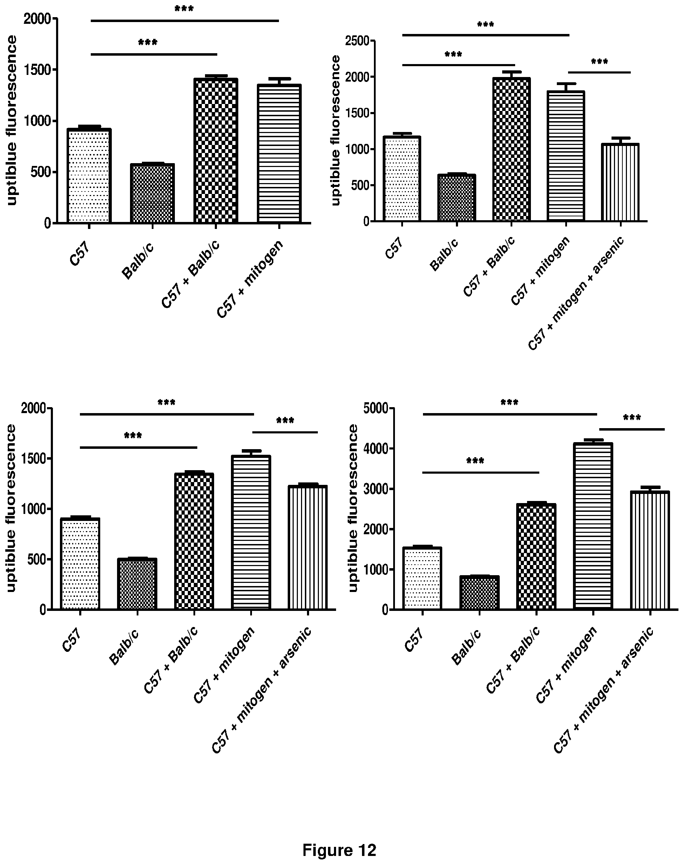

The results of these experiments on spleen cells for Controls, FeSO₄, HAuCl₂, Zn²⁺ salts (ZnSO₄ and ZnCl₂), Mn²⁺ salts (MnSO₄ and MnCl₂) and Cu²⁺ salts (CuSO₄ and CuCl₂) are illustrated in Figures 12 to 17, respectively. The statistically significant differences are represented on the figures as follows:
- control cells vs cells treated by As₂O₃
- control cells vs cells treated by cations only, and
- cells treated by As₂O₃ vs cells treated by As₂O₃ + cations.

*Validation of culture conditions:* Figure 12 shows the controls of proliferation measurement (4 independent tests): splenic cells from C57BL6 mice alone and splenic cells from BALB/c mice alone do not proliferate. In contrast, mixed cells show a lymphocyte reaction and a proliferation equal to that obtained in the presence of mitogen (CD3 5µg/ml/CD28 2µg/ml). Arsenic trioxide (1 µM) completely inhibits cell proliferation in the presence of mitogen.

*Note:* several tests have been performed to check the effect of a sequential deposit of As₂O₃ followed by metal ions (or the opposite metal ions + As₂O₃) or a solution of the two agents. No significant difference was found between the series of tests (data not shown).

### Effects of combinations of As₂O₃ and metal ions

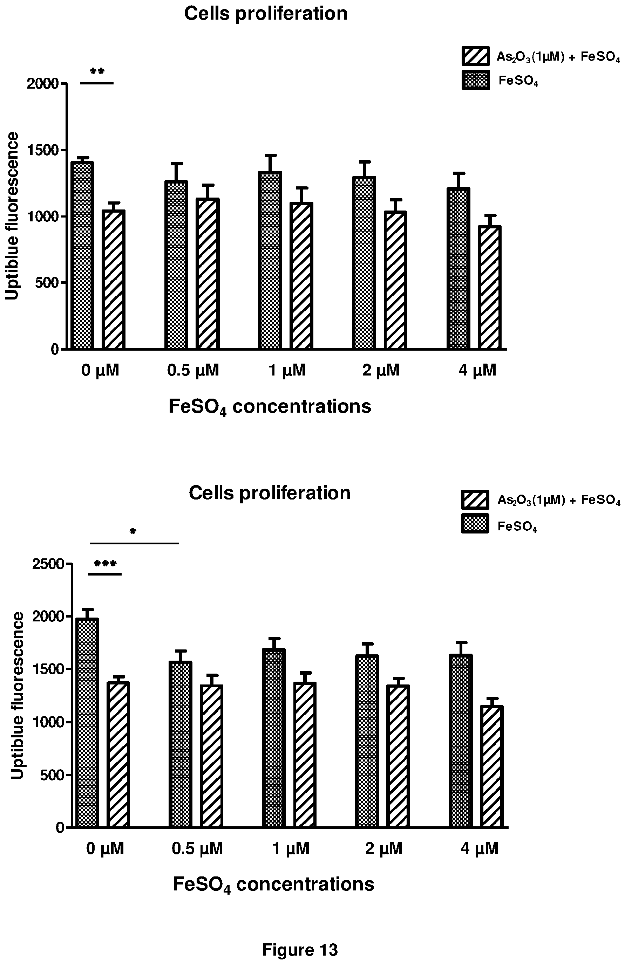

FeSO₄ does not have a significant effect on cell proliferation, either alone or with As₂O₃ (Figure 13).

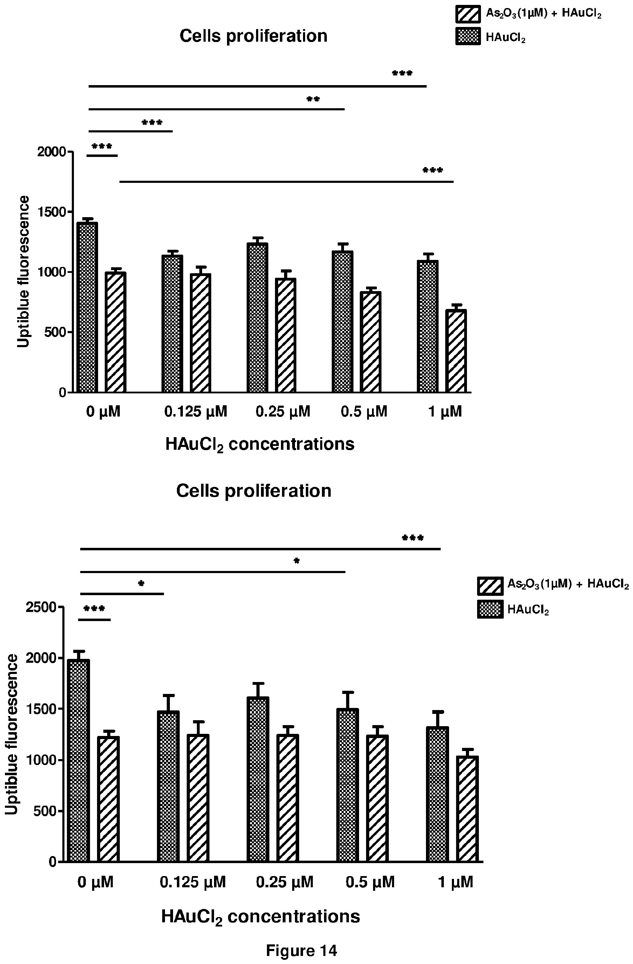

HAuCl₂ decreases the cell proliferation alone but this effect is limited at 1 µM with As₂O₃ and only for test 1 (p<0,001, Figure 14).

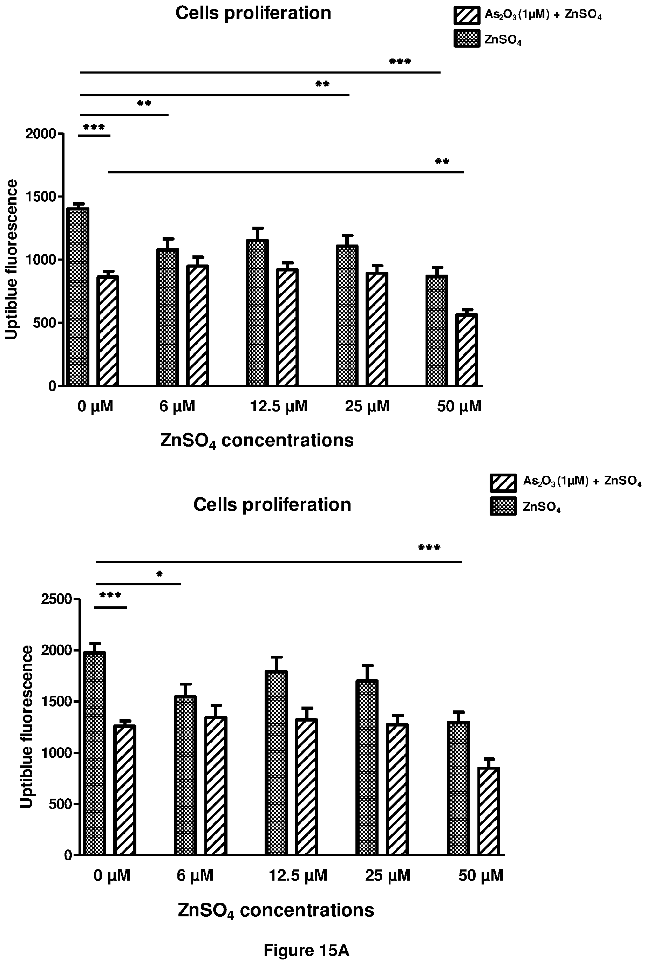

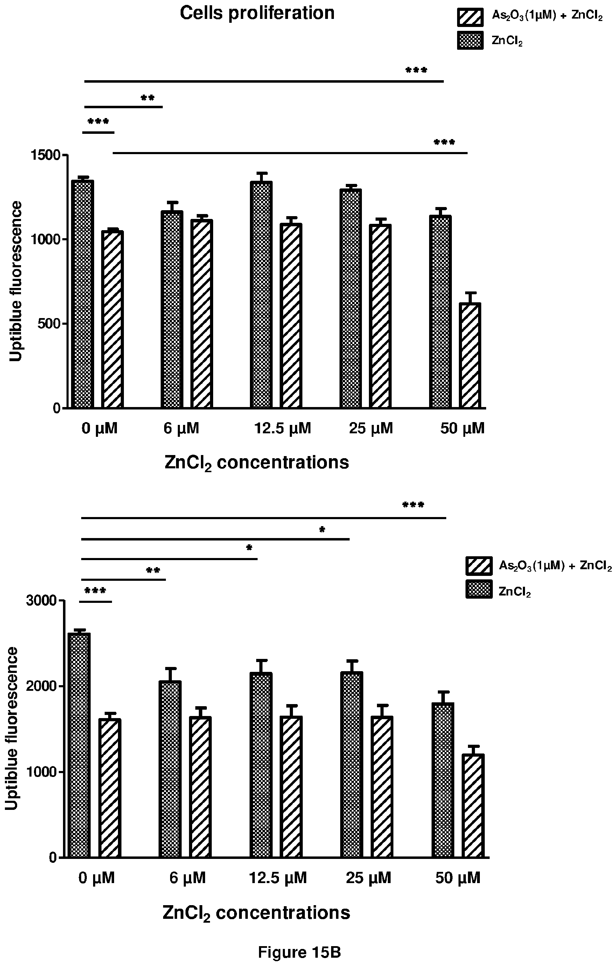

ZnSO₄ and ZnCl₂ decrease the cell proliferation alone, but this effect is limited at 50 µM with As₂O₃ and only for test 1 (p<0,01 and p<0,001 respectively, Figure 15).

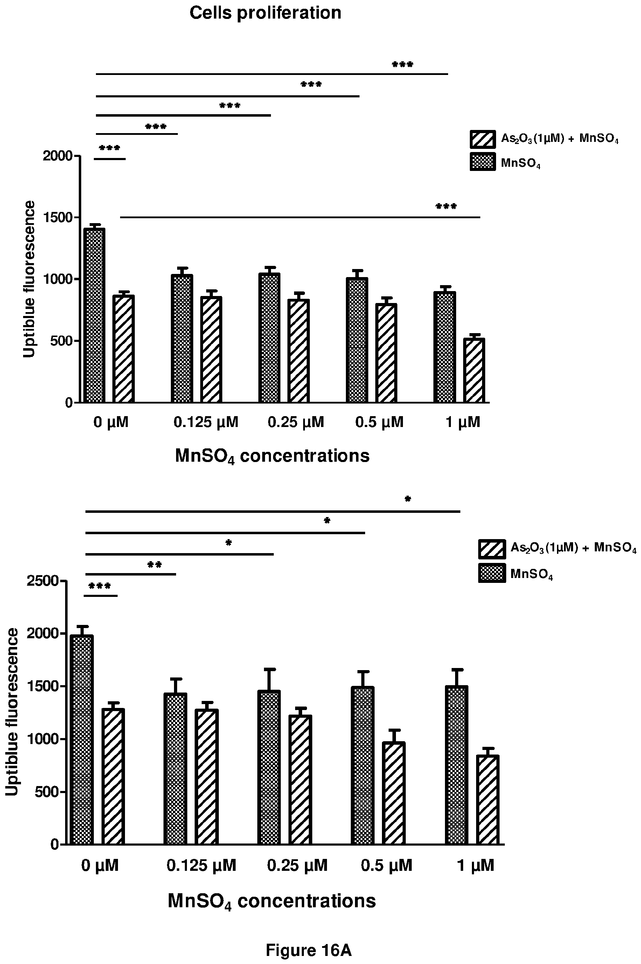

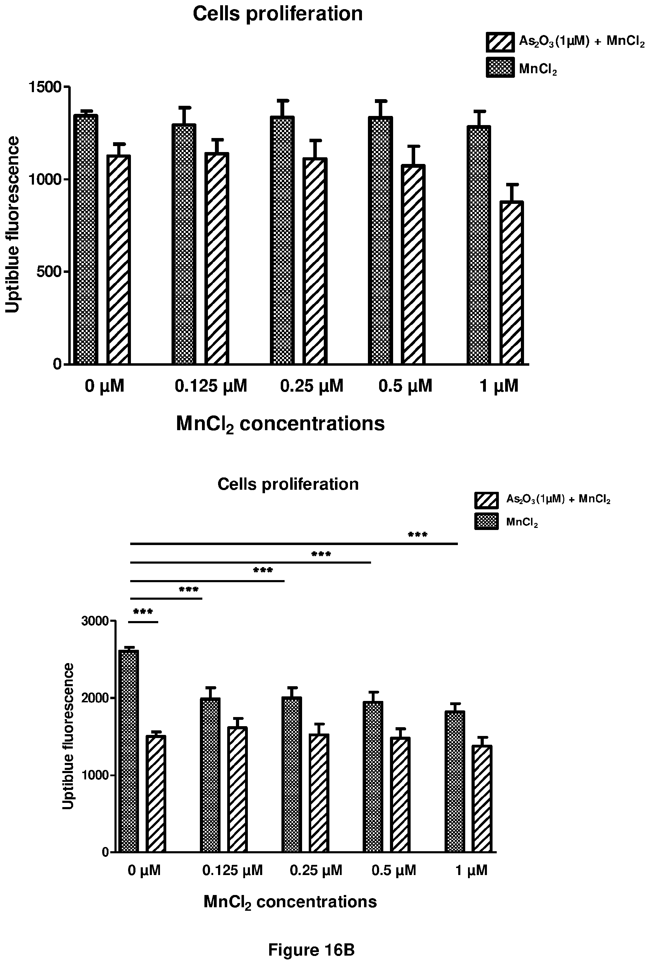

MnSO₄ and MnCl₂ significantly decrease the cell proliferation alone, but this effect is limited at 1 µM with As₂O₃ and only for test 1 (Figure 16).

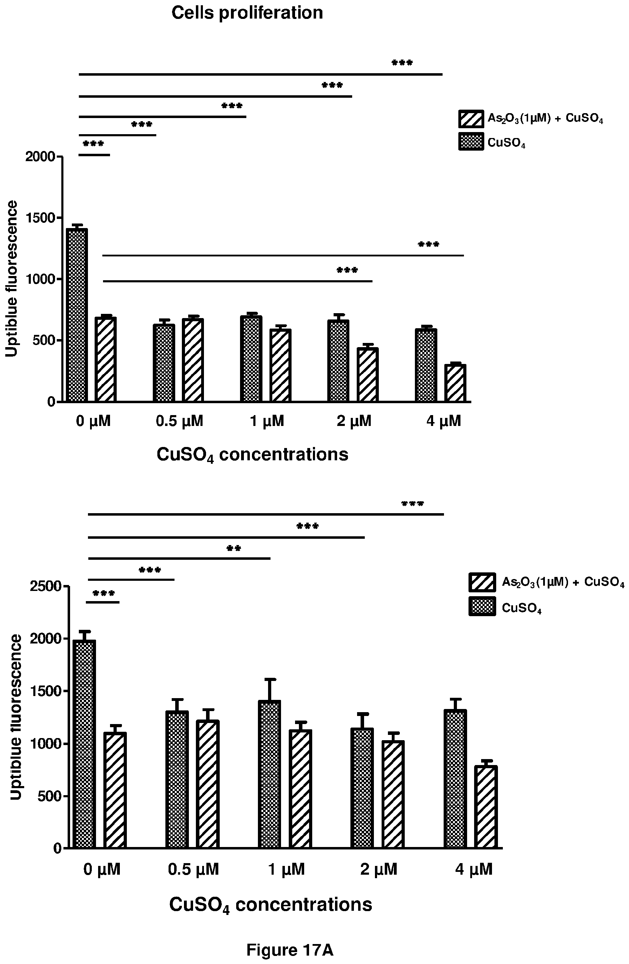

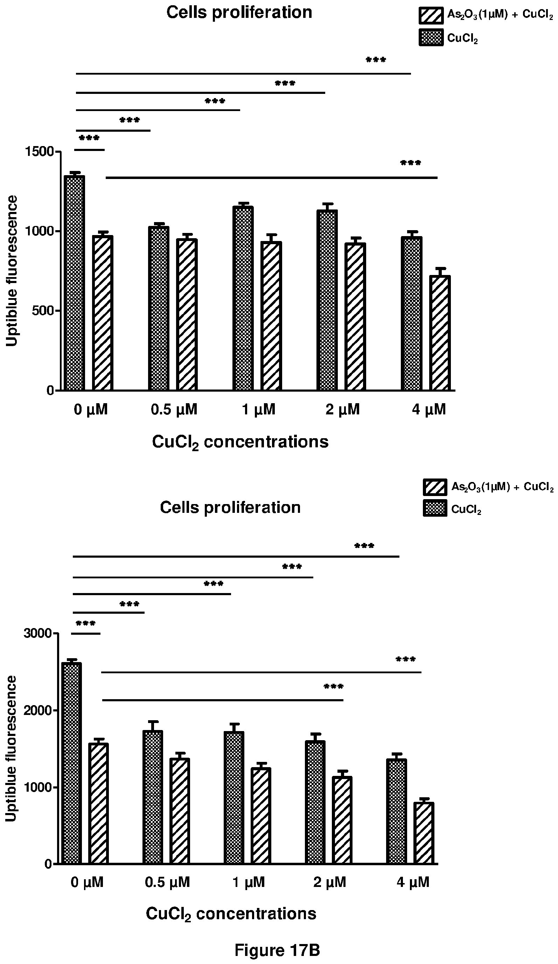

CuSO₄ and CuCl₂ significantly decrease the cell proliferation alone and this effect is significant at 4 µM or 2 and 4 µM with As₂O₃ (p<0.001, Figure 17).

Table 3 displays the significant results on cell proliferation decrease.

**Table 3**

| **Cations** | **Cell proliferation: Cations vs control** | **Cell proliferation: As₂O₃ + cations vs As₂O₃** |
|---|---|---|
| **FeSO₄ (2 tests)** | Not significant (test 1) 0.5 µM (test 2) | Not significant (tests 1 and 2) |
| **HauCl₂ (2 tests)** | 0.125, 0.50 and 1 µM (tests 1 and 2) | 1 µM (test 1) |
| | | Not significant (test 2) |
| **ZnSO₄ (2 tests)** | 6, 25 & 50 µM (test 1) | 50 µM (test 1) |
| | 6 & 50 µM (test 2) | Not significant (test 2) |
| **ZnCl₂ (2 tests)** | 6 & 50 µM (test 1) | 50 µM (test 1) |
| | 6, 12.5, 25 and 50 µM (test 2) | Not significant (test 2) |
| **MnSO₄ (2 tests)** | 0.125, 0.25, 0.50, 1 µM (tests 1 and 2) | 1 µM (test 1) |
| | | Not significant (test 2) |
| **MnCl₂ (2 tests)** | Not significant (test 1) | Not significant (tests 1 and 2) |
| | 0.125, 0.25, 0.50 and 1 µM (test 2) | |
| **CuSO₄ (2 tests)** | 0.5, 1, 2 and 4 µM (tests 1 and 2) | 2 and 4 µM (test 1) |
| | | Not significant (test 2) |
| **CuCl₂ (2 tests)** | 0.5, 1, 2 and 4 µM (tests 1 and 2) | 4 µM (test 1) |
| | | 2 and 4 µM (test 2) |

### Conclusion

Cu²⁺ is the most efficient metal ion for reducing the mixed lymphocyte reaction; changing the composition of the salt (CuSO₄ or CuCl₂) or the quality grade does not have any influence on the targeted effect.

### Example 6: Effects of combinations of As₂O₃ and CuCl₂ on H₂O₂ and GSH production and cell survival of A20 cells.

A20 is a murine lymphoma cell line derived from a spontaneous reticulum cell neoplasm of a Balb/C AnN mouse. In this study, arsenic was tested on a murine cell line of cancer cells to study its effect in the same way as for HL60 cells. Indeed, the future *in vivo* model being performed in mice, it was necessary to check the As₂O₃ effect on these murine cells *in vitro.*

In order to induce cell stress, the A20 cells (1×10⁵ cells/well/strain) were seeded in 96-well plates and incubated for 48 hours in complete medium with or without arsenic trioxide at 1 µM and with and without CuCl2 at 4 concentrations (0.5, 1, 2 and 4 µM) in an incubator at 37°C 5% CO₂.

The statistically significant differences are represented on the figures as follows:
- control cells vs cells treated by As₂O₃
- control cells vs cells treated by cations only
- cells treated by As₂O₃ vs cells treated by As₂O₃ + cations.

### Effects of combination of As₂O₃ and CuCl₂

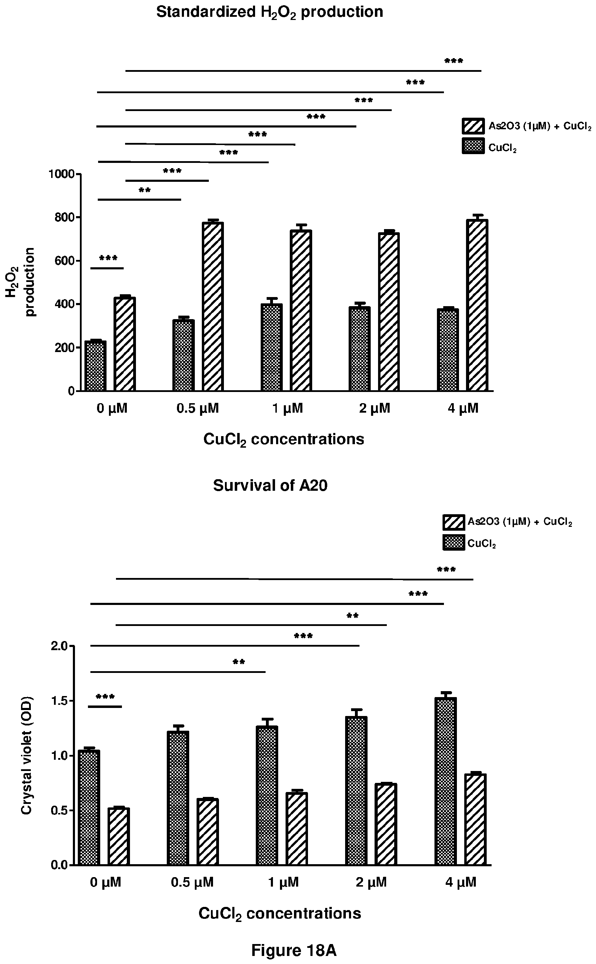

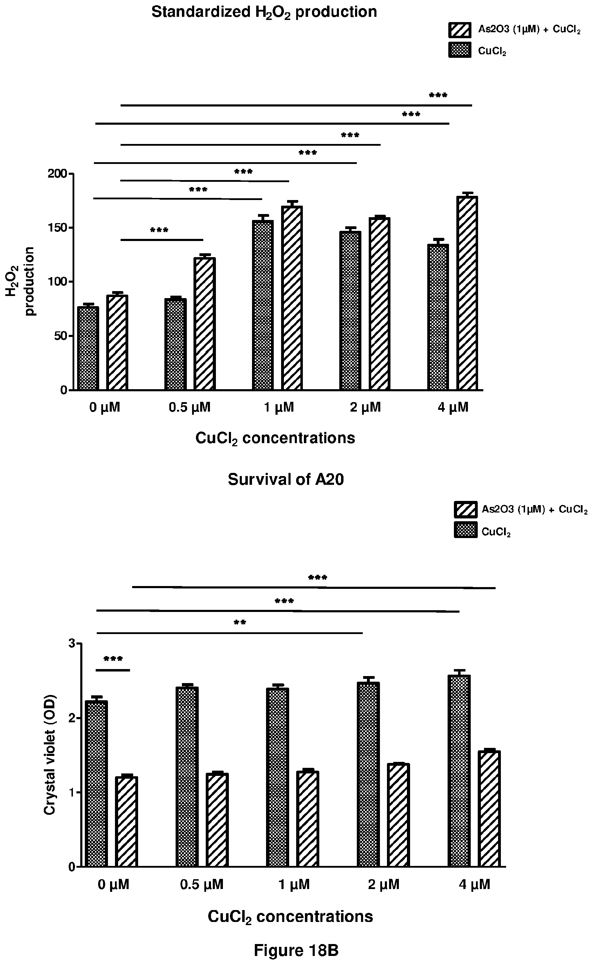

As₂O₃ at 1 µM significantly increased the H₂O₂ production and reduced the A20 viability (p<0.001, test 1, Figure 18A), but in test 2, As₂O₃ only significantly reduced the A20 viability (p<0.001, Figure 18B),

CuCl₂ alone increased the H₂O₂ production at any of the 4 concentrations tested (test 1 p<0.001) and at 3 higher concentrations (test 2, p<0.001). CuCl₂ alone apparently increased the A20 viability at 3 or 2 higher concentrations tested (Figure 18).

At any of the 4 tested concentrations, CuCl₂ increased the effect induced by As₂O₃ on H₂O₂ production (Tests 1 and 2, Figure 18, left-hand panels). In the presence of As₂O₃, CuCl₂ slightly but significantly increased A20 viability at higher concentrations (Test 1: 2 and 4 µM and Test 2: 4 µM; Figure 18, right-hand panels).

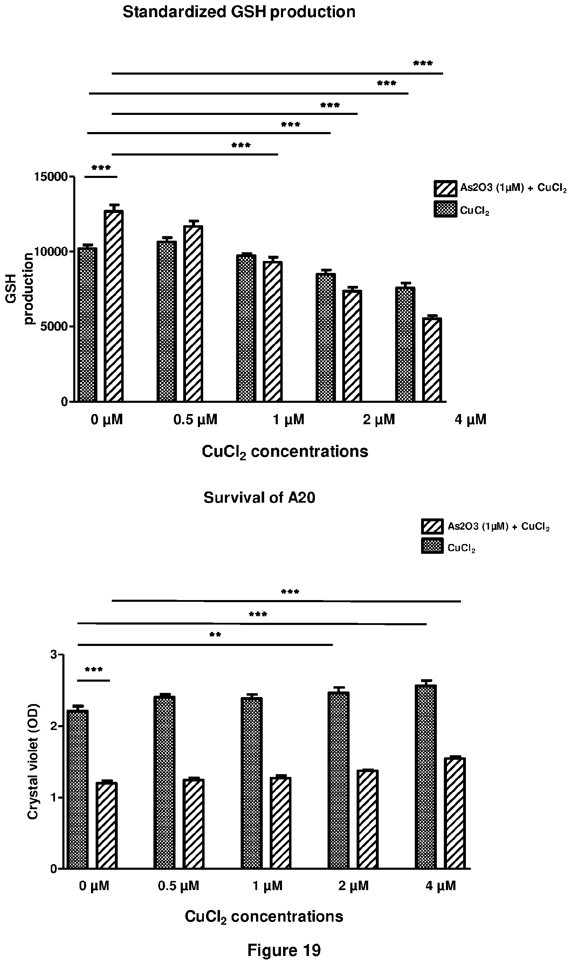

As₂O₃ at 1 µM significantly increased the GSH production and significantly reduced A20 cells viability (p<0.001, test 1, Figure 19).

CuCl₂ alone significantly decreased the GSH production and increased A20 cells viability at 2 and 4 µM (test 1; Figure 19). In the presence of As₂O₃ , CuCl₂ significantly decreased arsenic-induced GSH production at higher concentrations and increased A20 cells viability only at 4 µM (test 1; Figure 19).

Tables 4 and 5 display the significant results on H₂O₂ and GSH production and A20 viability.

**Table 4**

| **Cations** | **H₂O₂ Production: cations vs control** | **H₂O₂ Production: As₂O₃ + cations vs As₂O₃** | **Cell survival: cations vs control** | **Cell survival: As₂O₃ + cations vs As₂O₃** |
|---|---|---|---|---|
| **CuCl₂ (2 tests)** | 0.5, 1, 2 and 4 µM (test 1) | 0.5, 1, 2 and 4 µM (tests 1 and 2) | 1, 2 and 4 µM (test 1) | 2 and 4 µM (test 1) |
| | 1, 2 and 4 µM (test 2) | | 2 and 4 µM (test 2) | 4 µM (test 2) |

**Table 5**

| **Cations** | **GSH Production: cations vs control** | **GSH Production: As₂O₃ + cations vs As₂O₃** | **Cell survival: cations vs control** | **Cell survival: As₂O₃ + cations vs As₂O₃** |
|---|---|---|---|---|
| **CuCl₂ (1 test)** | 2 and 4 µM | 1, 2 and 4 µM | 2 and 4 µM | 4 µM |

### Conclusion

In the presence of As₂O₃, CuCl₂ significantly increased the H₂O₂ production and decreased the GSH production without any significant effect on A20 cell survival.

### Example 7: Effects of AsI₃ on H₂O₂ and GSH production and cell survival of HL60 cells

In order to induce cell stress, the HL60 cells (5×10⁵ cells/well/strain) were seeded in 96-well plates (Falcon, Corning, reference: 353077) and incubated for 48 hours in complete medium with or without AsI₃ at several concentrations: 0.1; 0.5; 1; 5 and 10µM in an incubator at 37°C 5% CO₂.

### Effects of AsI₃ on HL60-cells:

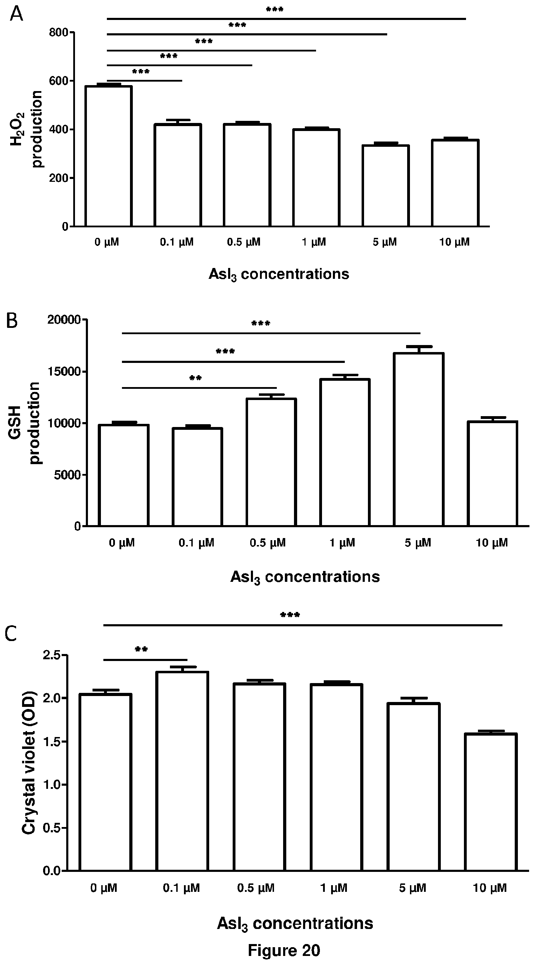

Figures 20 to 23 show the effect of AsI₃ at increasing concentrations on the production of H₂O₂ and GSH, and cell viability (1 test).

AsI₃ significantly reduced H₂O₂ production at all tested concentrations (p<0.001, Figure 20A).

Asl₃ significantly increased GSH production at 0.1, 1 and 5 µM (p<0.01, p<0.001 and p<0.001 respectively, Figure 20B).

Asl₃ significantly reduced HL60 cells viability at 10 µM (p<0.001, Figure 20C).

### Conclusion

The dose of 1 µM of Asl₃ was selected to test the effects of combinations with CuCl₂ on H₂O₂ production and HL60-survival.

### Example 8: Effects of combinations of Asl₃ and CuCl₂ on H₂O₂ production and cell survival of HL60 cells

In order to induce cell stress, the HL60 cells (5×10⁵ cells/well/strain) were seeded in 96-well plates and incubated for 48 hours in complete medium with or without Asl₃ at 1 µM and with and without CuCl₂ at 0.5, 1, 2 and 4 µM in an incubator at 37°C 5% CO₂.

### Effects of combination of AsI₃ and CuCl₂:

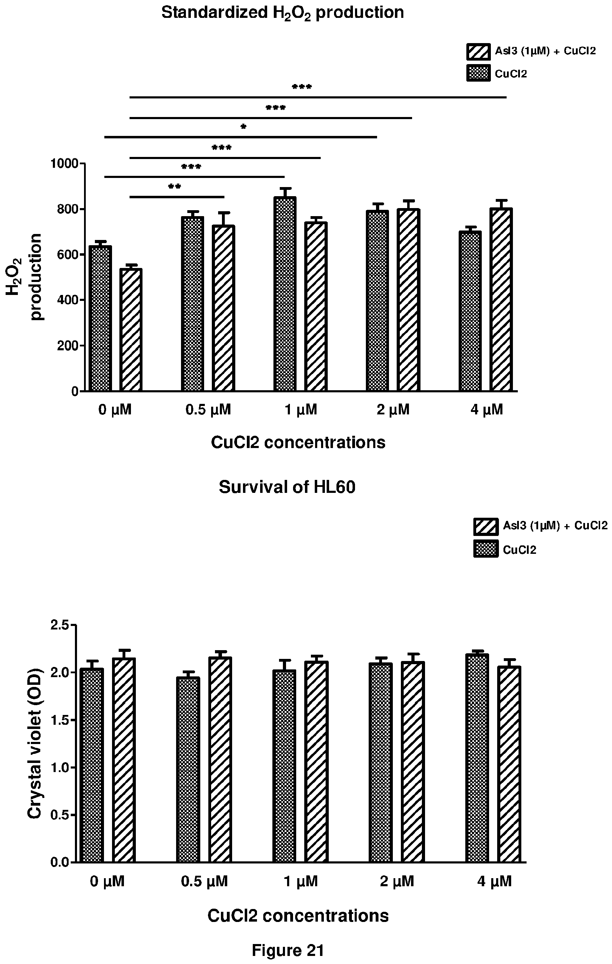

Asl₃ at 1 µM has not effect on H₂O₂ production and cell survival (Figure 21).

Whatever the tested concentration, CuCl₂ significantly increased the effect of Asl₃ (at 1µM) on H₂O₂ production (Figure 21, left-hand panel). CuCl₂ had no effect on cell survival in presence of Asl₃ (Figure 21, right-hand panel).

### Conclusion:

In the presence of Asl₃ (1µM), there is already a significant potentializing effect of CuCl₂ on H₂O₂ production, although it appears to be lower than that measured in the presence of As₂O₃.

### Example 9: Effects of Asl₃ on the proliferative properties of C57BI6 splen cells during a mixed lymphocytic reaction (MLR)

*In vitro* cell proliferation assays were performed on spleen cells from C57BL6 mice in co-culture with previously irradiated (30 Gray) so-called "stimulating" splenic cells from BALB/c mice.

For the proliferation tests, the cells (6 × 10⁵ cells/well/strain) were seeded in 96-well plates (Falcon, Corning, reference: 353077) and incubated for 48 hours in complete medium supplemented or not, with arsenic trioxide (As₂O₃) at 1 µM or with arsenic triiodide (Asl₃) at 1 µM in presence of mitogen or with copper at increasing concentrations, from 0.5 to 4 µM CuCl₂.

Cell proliferation was determined by a spectrofluorimeter assay after 24 hours of incubation of the cells with 10% UptiBlue (20 µL in 200 µL of medium).

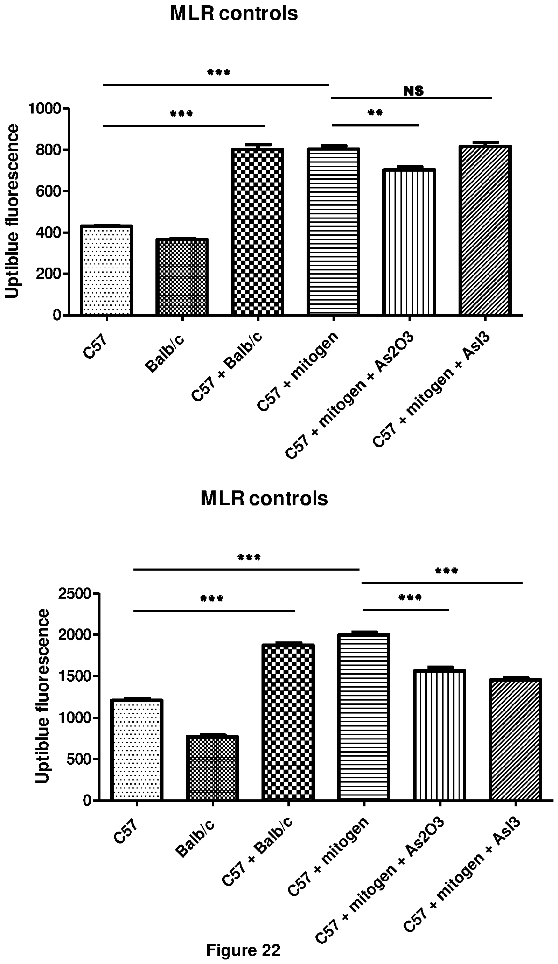

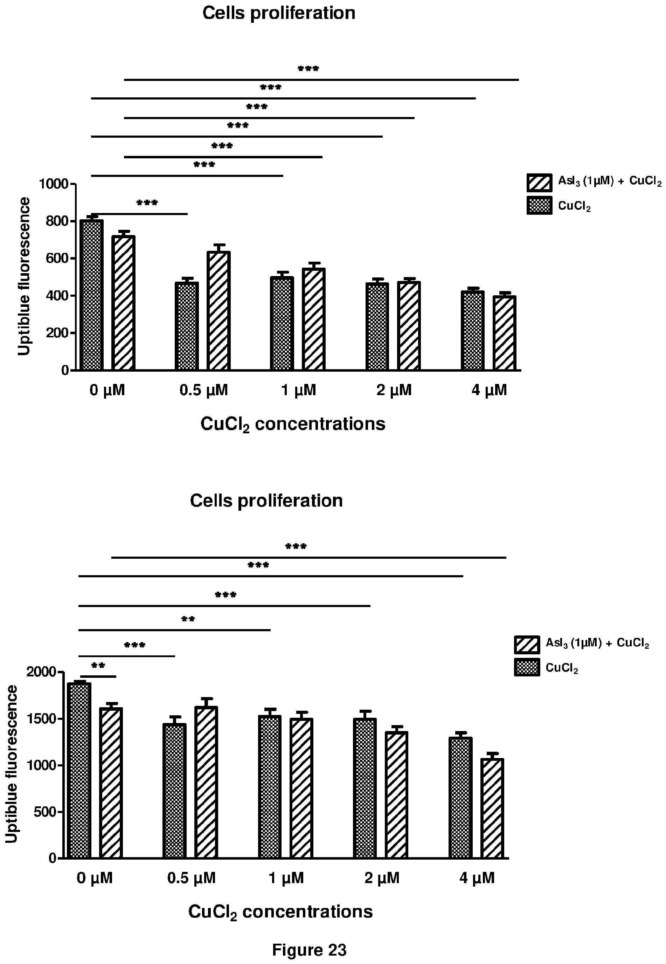

The results of arsenic trioxide (As₂O₃) at 1 µM or with arsenic triiodide (Asl₃) at 1 µM on cell proliferation in the presence of mitogen are illustrated in Figure 22. Asl₃ is only significantly active on test 2 (Figures 22 and 23, right-hand panels).

The results of these experiments on spleen cells for CuCl₂ with more or less arsenic triiodide (Asl₃) are illustrated in Figure 23.

In the presence of Asl₃, CuCl₂ significantly decreased the proliferation of C57BL6 spleen cells at 1, 2 and 4 µM (test 1) and at 4 µM (test 2).

### Conclusion:

Asl₃ is active, but slightly less than As₂O₃ and the synergy with CuCl₂ seems reduced, probably indicating that the important feature is the concentration in the As ion.

### Example 10: Effects of As₂O₃ and CuCl₂ in a mice model of Graft versus Host Disease (GvHD)

The association of arsenic trioxide (As₂O₃) and copper molecules (CuCl₂) was then evaluated in a well-established murine model of chronic sclerodermatous Graft versus Host Disease (Arsenic Trioxide Prevents Murine Sclerodermatous Graft-versus-Host Disease, Kavian *et* al., 2012). In this model, mice (female BALB/c mice-H-2d) previously sub-lethally irradiated with 7.5 Grays from a Gammacel source are grafted with bone marrow (1×10⁶ cells) and splenocytes (2×10⁶ cells) from mice presenting a low immune compatibility (male B10.D2 mice-H-2b).

7 days after transplantation, the mice received intraperitoneal injections of arsenic trioxide associated or not with copper chloride ("copper"), 5 times a week for 5 weeks. An irradiation control group was carried out to verify the irradiation efficacy and that the graft had succeeded in the recipient mice. A syngeneic group was grafted with splenocytes and bone marrow from mice of the same genetic background. This control group should not develop chronic GvHD. Once a week, a clinical assessment, scoring signs such as alopecia, weight loss, diarrhea, vasculitis allowed to evaluate the progression of the disease.

**Table 6: Experimental groups, with the strains of donor and recipient mice and the number of mice per group**

| Groups | Recipients | Donor | Number of mice |
|---|---|---|---|
| Irradiation control | BALB/c | Ø | 3 |
| Syngeneic | BALB/c | BALB/c | 9 |
| Allogeneic | BALB/c | B10.D2 | 10 |
| Allogeneic + As₂O₃ 2,5 µg/g | BALB/c | B10.D2 | 10 |
| Allogeneic + As₂O₃ 2,5 µg/g + CuCl₂ 10 µg/g | BALB/c | B10.D2 | 10 |
| Allogeneic + CuCl₂ 10 µg/g | BALB/c | B10.D2 | 7 |
| Allogeneic + As₂O₃ 2,5 µg/g + CuCl₂ 2,5 µg/g | BALB/c | B10.D2 | 10 |
| Allogeneic + CuCl₂ 2,5 µg/g | BALB/c | B10.D2 | 7 |
| Allogeneic + As₂O₃ 5 µg/g | BALB/c | B10.D2 | 7 |

Initial protocol: After 5 weeks, the mice should have been euthanized in order to evaluate the effect of the association of copper with arsenic trioxide on the organs affected by chronic GvHD (skin, lung, liver ...).

Protocol adjustment: Following a significant loss of mice in the copper-treated groups, the injections had to be stopped at the end of day 4. The treatment was resumed the following week on day 8 at a different rate until the end of the 5 weeks of treatment. Copper was then injected only twice a week instead of 5 in mice receiving copper, while mice treated with arsenic alone or combined to copper still received 5 injections of ATO per week. Under these new treatment conditions, no more copper-treated mice died.

The number of mice being greatly reduced, we decided to collect only the blood of the mice in order to perform the transaminase tests, but the organs were not harvested. These experiments show that Copper needs to be reduced to abolish its toxic effect on mice, at least when it is administered via the intraperitoneal route.

**Table 7: Number of live mice at the end of the experiment compared to the initial number of mice**

| Groups | Number of mice at the beginning | Number of mice at the end |
|---|---|---|
| Irradiation control | 3 | 0 |
| Syngeneic | 9 | 8 |
| Allogeneic | 10 | 9 |
| Allogeneic + As₂O₃ 2,5 µg/g | 10 | 9 |
| Allogeneic + As₂O₃ 2,5 µg/g + CuCl₂ 10 µg/g | 10 | 2 |
| Allogeneic + CuCl₂ 10 µg/g | 7 | 5 |
| Allogeneic + As₂O₃ 2,5 µg/g + CuCl₂ 2,5 µg/g | 10 | 3 |
| Allogeneic + CuCl₂ 2,5 µg/g | 7 | 2 |
| Allogeneic + As₂O₃ 5 µg/g | 7 | 5 |

The results of this experiment are shown in Table 8 below and in Figures 24 to 27.

**Table 8: Results of the experiment (alopecia)**

| Groups | Number of mice | Number of mice with alopecia | Percentage of mice with alopecia |
|---|---|---|---|
| Syngeneic | 8 | 0 | 0% |
| Allogeneic | 9 | 7 | **77 %** |
| Allogeneic + As₂O₃ 2.5 µg/g | 9 | 3 | 33 % |
| Allogeneic + As₂O₃ 2.5 µg/g + CuCl₂ 2.5 µg/g | 3 | 0 | 0% |
| Allogeneic + CuCl₂ 2.5 µg/g | 2 | 1 | 50 % |
| Allogeneic + As₂O₃ 2.5 µg/g + CuCl₂ 10 µg/g | 2 | 0 | 0% |
| Allogeneic + CuCl₂ 10 µg/g | 5 | 0 | 0% |
| Allogeneic + As₂O₃ 5 µg/g | 5 | 1 | 20 % |

Even though the number of mice per group is insufficient for significant statistics, we can still observe that none of the mice receiving a combined treatment with As₂O₃ 2.5 µg/g and CuCl₂ (2.5 or 10 µg/g) developed alopecia, which is better than mice receiving only As₂O₃ 2.5 µg/g (33% alopecia) and even better of mice receiving As₂O₃ 5 µg/g (20% alopecia) (Figure 24). This confirms that the effects of arsenic are potentiated when co-administered with Copper.

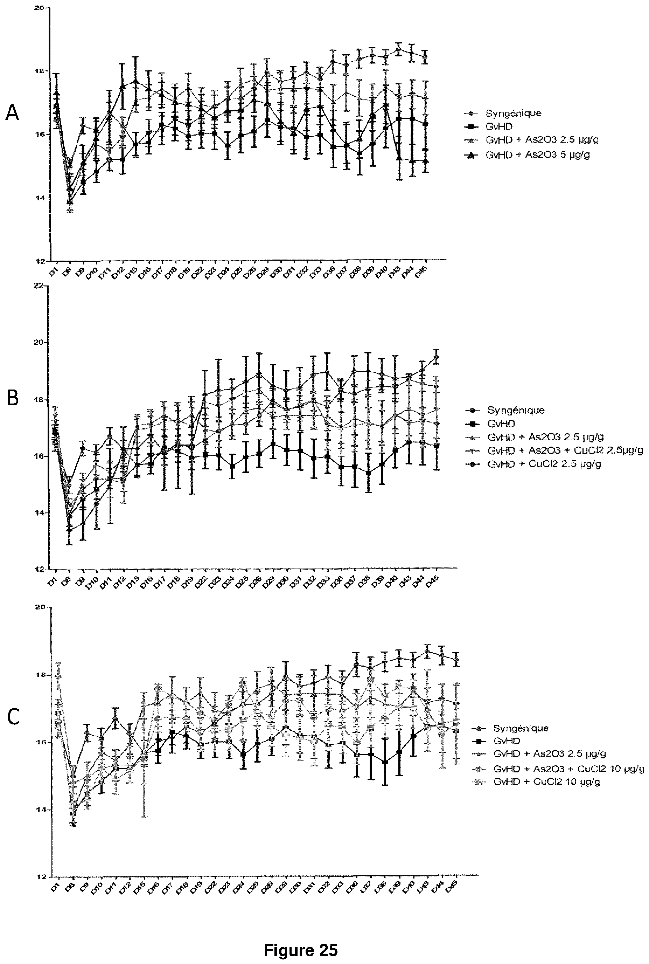

Concerning the weights of the mice, at the end of the treatment, there was no obvious difference between the groups even if the mice of the syngeneic group seemed to have a higher weight than the mice of the other groups (figure 25).

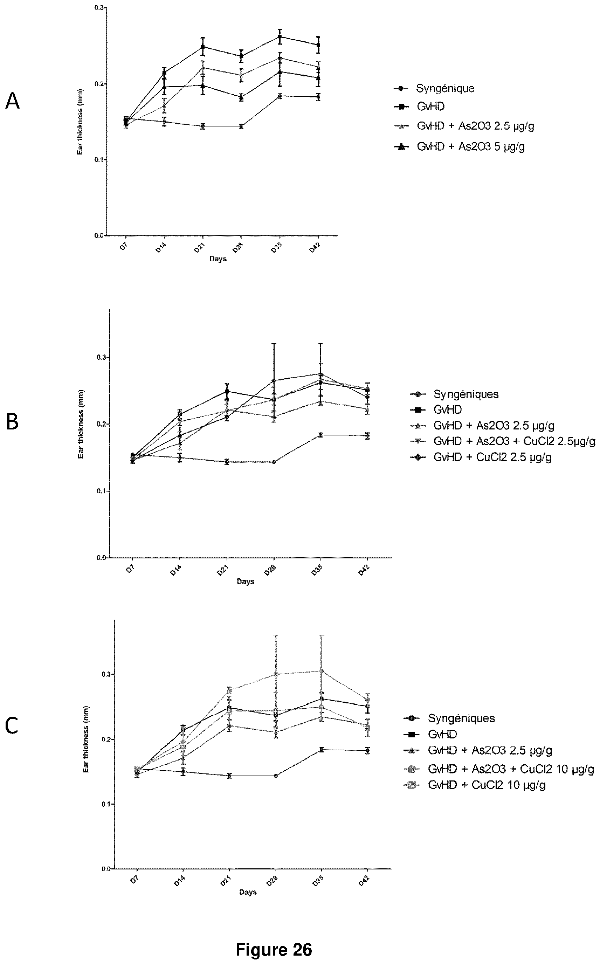

Mice in the allogeneic group show an increase in ear thickness (vasculitis) compared to mice in the syngeneic group. We also observe that in the allogeneic group + As₂O₃ 2.5 µg/g, vasculitis was reduced and in the allogenic group + 5 µg/g, it decreased even more until reaching the same values as the ones of the syngeneic group. (Figure 26)

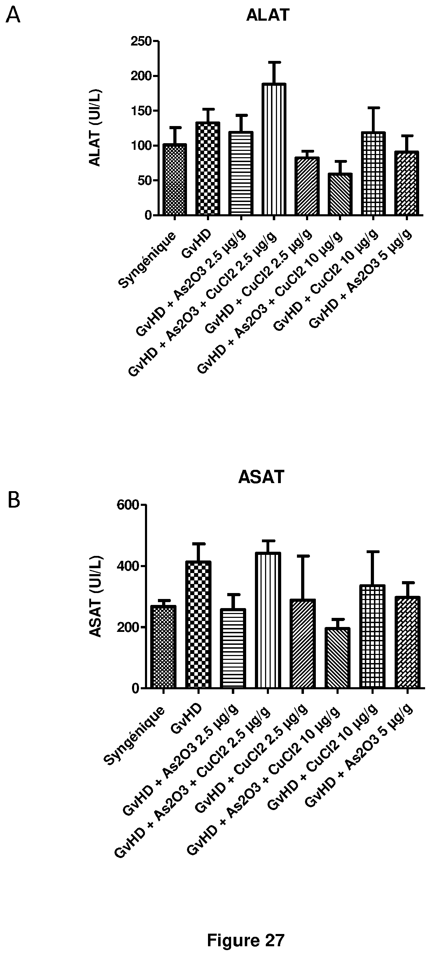

Regarding the hepatic balance of animals, results obtained for sera transaminases are not consistent. This may be due to a variable toxic effect of our repeated intraperitoneal injections of arsenic trioxide and copper, liver being exposed to variable amounts depending on the particular site of each injection, and thus variable deleterious effects (Figure 27).

### Example 11: Effects of As₂O₃ and CuCl₂ in a mice model of Graft versus Host Disease (GvHD), with a lower dosage of CuCl₂

Following these results, we decided to perform a copper cytotoxicity assessment. To this aim, we injected a lower dose of copper (0.2 and 0.5 µg/g) 5 times a week for 5 weeks to a new series of BALB/c mice. We observed the mice every day, monitored their weight and followed their survival to evaluate copper toxicity.

After 5 weeks, there was no death and the mean weights were similar between the control group receiving PBS and the groups receiving copper at 0.2 and 0.5 µg/g. The dosage of 0.5 µg/g was thus chosen for a new set of experiments, using the same protocol as described in Example 10, but with the conditions described in Table 9 below.

**Table 9: Experimental groups, with the strains of donor and recipient mice and the number of mice per group**

| Groups | Recipients | Donor | Number of mice |
|---|---|---|---|
| Irradiation control | BALB/c | Ø | 3 |
| Syngeneic | BALB/c | BALB/c | 7 |
| Allogeneic | BALB/c | B10.D2 | 7 |
| Allogeneic + As₂O₃ 2,5 µg/g | BALB/c | B10.D2 | 10 |
| Allogeneic + As₂O₃ 2,5 µg/g + CuCl₂ 0.5 µg/g | BALB/c | B10.D2 | 10 |
| Allogeneic + CuCl₂ 0.5 µg/g | BALB/c | B10.D2 | 10 |
| Allogeneic + As₂O₃ 5 µg/g | BALB/c | B10.D2 | 10 |

## Claims

1. A composition comprising arsenic trioxide and Cu²⁺ ions, for use in treating a disease selected from the group consisting of a neoplastic disease, an autoimmune disease, an inflammatory disease and various forms of multiple sclerosis (MS), wherein the amount of arsenic trioxide in one daily dose is between 0.01 and 0.10 mg/kg/day and wherein the amount of Cu2+ in one daily dose is between 0.05 µmol/kg and 2 µmol/kg of Cu²⁺.

2. The composition for use according to claim 1, wherein the Cu²⁺ ions are in the form of a salt selected from the group consisting of copper sulfate and copper(II) chloride.

3. The composition for use according to claim 1 or claim 2, wherein one daily dose comprises between 0.01 and 0.05 mg/kg/day of arsenic trioxide.

4. The composition for use according to any one of claims 1 to 3, wherein one daily dose comprises between 0.06 µmol/kg and 2 µmol/kg of Cu²⁺, preferably between 0.3 µmol/kg and 1.1 µmol/kg of Cu²⁺.

5. The composition for use according to any one of claims 1 to 4, wherein the disease is selected from the group consisting of acute promyelocytic leukemia systemic lupus erythematosus (SLE), various forms of multiple sclerosis (MS), Sjögren syndrome, rheumatoid arthritis, Crohn's disease, graft versus host disease (GvHD), myelocytic leukemia, chronic myeloid leukemia, chronic lymphocytic leukemia, malignant glioma, myelodysplastic syndrome, multiple myeloma, and liver, breast, pancreas and prostate cancers.

6. A combination of a Cu²⁺ salt and an arsenic compound, for use in the treatment of a disease selected from the group consisting of a neoplastic disease, an autoimmune disease, an inflammatory disease and various forms of multiple sclerosis (MS), wherein said arsenic compound and said Cu²⁺ salt are administered to a patient sequentially, and wherein the amount of As element in one daily dose is between 0.1 µmol/kg and 1.6 µmol/kg and the amount of Cu²⁺ in one daily dose is between 0.05 µmol/kg and 2 µmol/kg.

7. A combination of a Cu²⁺ salt and arsenic trioxide, for use in the treatment of a disease selected from the group consisting of a neoplastic disease, an autoimmune disease, an inflammatory disease and various forms of multiple sclerosis (MS), wherein said arsenic trioxide and said Cu²⁺ salt are administered to a patient simultaneously or sequentially, and wherein the amount of arsenic trioxide in one daily dose is between 0.01 and 0.10 mg/kg/day and the amount of Cu²⁺ in one daily dose is between 0.05 µmol/kg and 2 µmol/kg.

8. Arsenic trioxide, for use in the treatment of a disease selected from the group consisting of a neoplastic disease, an autoimmune disease, an inflammatory disease and various forms of multiple sclerosis (MS), wherein said arsenic trioxide is administered to a patient in combination with a Cu²⁺ salt, at a daily dose such that the amount of arsenic trioxide in one daily dose is between 0.01 and 0.10 mg/kg/day and the amount of Cu²⁺ in one daily dose is between 0.05 µmol/kg and 2 µmol/kg.

9. The combination of claim 7 or the arsenic trioxide of claim 8, for the use of claim 7 or claim 8, wherein the amount of arsenic trioxide in one daily dose is between 0.01 and 0. 05 mg/kg/day.

10. The combination or the arsenic trioxide of any one of claims 6 to 9, for the use of any one of claims 6 to 9, wherein the Cu²⁺ salt is copper sulfate or copper(II) chloride.

11. The combination or the arsenic trioxide of any one of claims 6 to 10, for the use of any one of claims 6 to 10, wherein said disease is selected from the group consisting of acute promyelocytic leukemia, systemic lupus erythematosus (SLE), multiple sclerosis (MS), Sjögren syndrome, rheumatoid arthritis, Crohn's disease, graft versus host disease (GvHD), myelocytic leukemia, chronic myeloid leukemia, chronic lymphocytic leukemia, malignant glioma, myelodysplastic syndrome, multiple myeloma and liver, breast, pancreas and prostate cancer.

12. A composition consisting essentially of a Cu²⁺ salt, for use in the treatment of a disease selected from the group consisting of a neoplastic disease, an autoimmune disease, an inflammatory disease and various forms of multiple sclerosis(MS), in a patient receiving an arsenic compound such that the amount of As element in one daily dose is between 0.1 µmol/kg and 1.6 µmol/kg, wherein one daily dose of the composition comprises between 0.05 µmol/kg and 2 µmol/kg of Cu²⁺ and wherein said Cu²⁺ salt potentiates the effects of the arsenic compound.

13. The combination for use of claim 6 or the composition for use of claim 12, wherein the arsenic compound is selected from the group consisting of As₂O₃, Asl₃, As₂O₅, As₄O₆, As₂S₂, As₂S₃, As₂S₅, As₄S₄ and mixtures thereof, preferably arsenic trioxide or arsenic triiodide.

14. The composition of claim 12, for the use of claim 12, wherein said disease is selected from the group consisting of acute promyelocytic leukemia, systemic lupus erythematosus (SLE), various forms of multiple sclerosis (MS), Sjögren syndrome, rheumatoid arthritis, Crohn's disease, chronic graft versus host disease (GvHD), myelocytic leukemia, chronic myeloid leukemia, chronic lymphocytic leukemia, malignant glioma, myelodysplastic syndrome, multiple myeloma, and liver, breast and prostate cancers.

## Patentansprüche

1. Zusammensetzung, die Arsentrioxid und Cu²⁺-Ionen umfasst, zur Verwendung bei der Behandlung einer Erkrankung, ausgewählt aus der Gruppe bestehend aus einer neoplastischen Erkrankung, einer Autoimmunerkrankung, einer entzündlichen Erkrankung und verschiedenen Formen von Multipler Sklerose (MS), wobei die Menge an Arsentrioxid in einer Tagesdosis zwischen 0,01 und 0,10 mg/kg/Tag beträgt, und wobei die Menge an Cu2+ in einer Tagesdosis zwischen 0,05 µmol/kg und 2 µmol/kg Cu²⁺ beträgt.

2. Zusammensetzung zur Verwendung nach Anspruch 1, wobei die Cu²⁺-Ionen in der Form eines Salzes vorliegen, ausgewählt aus der Gruppe bestehend aus Kupfersulfat und Kupfer(II)-chlorid.

3. Zusammensetzung zur Verwendung nach Anspruch 1 oder Anspruch 2, wobei eine Tagesdosis zwischen 0,01 und 0,05 mg/kg/Tag Arsentrioxid umfasst.

4. Zusammensetzung zur Verwendung nach einem der Ansprüche 1 bis 3, wobei eine Tagesdosis zwischen 0,06 µmol/kg und 2 µmol/kg Cu²⁺, vorzugsweise zwischen 0,3 µmol/kg und 1,1 µmol/kg Cu²⁺ umfasst.

5. Zusammensetzung zur Verwendung nach einem der Ansprüche 1 bis 4, wobei die Erkrankung ausgewählt ist aus der Gruppe bestehend aus akuter Promyelozytenleukämie, systemischem Lupus erythematodes (SLE), verschiedenen Formen von Multipler Sklerose (MS), Sjögren-Syndrom, rheumatoider Arthritis, Morbus Crohn, Transplantat-gegen-Wirt-Erkrankung (Graft versus Host Disease, GvHD), Myelozytenleukämie, chronischer myeloischer Leukämie, chronischer Lymphozytenleukämie, malignem Gliom, myelodysplastischem Syndrom, multiplem Myelom, und Leber-, Brust-, Bauchspeicheldrüsen- und Prostatakrebs.

6. Kombination aus einem Cu²⁺-Salz und einer Arsenverbindung zur Verwendung bei der Behandlung einer Erkrankung, ausgewählt aus der Gruppe bestehend aus einer neoplastischen Erkrankung, einer Autoimmunerkrankung, einer entzündlichen Erkrankung und verschiedenen Formen von Multipler Sklerose (MS), wobei die Arsenverbindung und das Cu²⁺-Salz einem Patienten nacheinander verabreicht werden, und wobei die Menge des Elements As in einer Tagesdosis zwischen 0,1 µmol/kg und 1,6 µmol/kg beträgt, und die Menge an Cu²⁺ in einer Tagesdosis zwischen 0,05 µmol/kg und 2 µmol/kg beträgt.

7. Kombination aus einem Cu²⁺-Salz und Arsentrioxid zur Verwendung bei der Behandlung einer Erkrankung, ausgewählt aus der Gruppe bestehend aus einer neoplastischen Erkrankung, einer Autoimmunerkrankung, einer entzündlichen Erkrankung und verschiedenen Formen von Multipler Sklerose (MS), wobei das Arsentrioxid und das Cu²⁺-Salz einem Patienten gleichzeitig oder nacheinander verabreicht werden, und wobei die Menge an Arsentrioxid in einer Tagesdosis zwischen 0,01 und 0,10 mg/kg/Tag beträgt, und die Menge an Cu²⁺ in einer Tagesdosis zwischen 0,05 µmol/kg und 2 µmol/kg beträgt.

8. Arsentrioxid zur Verwendung bei der Behandlung einer Erkrankung, ausgewählt aus der Gruppe bestehend aus einer neoplastischen Erkrankung, einer Autoimmunerkrankung, einer entzündlichen Erkrankung und verschiedenen Formen von Multipler Sklerose (MS), wobei das Arsentrioxid einem Patienten in Kombination mit einem Cu²⁺-Salz in einer Tagesdosis derart verabreicht wird, dass die Menge an Arsentrioxid in einer Tagesdosis zwischen 0,01 und 0,10 mg/kg/Tag beträgt, und die Menge an Cu²⁺ in einer Tagesdosis zwischen 0,05 µmol/kg und 2 µmol/kg beträgt.

9. Kombination nach Anspruch 7 oder Arsentrioxid nach Anspruch 8 zur Verwendung nach Anspruch 7 oder Anspruch 8, wobei die Menge an Arsentrioxid in einer Tagesdosis zwischen 0,01 und 0,05 mg/kg/Tag beträgt.

10. Kombination oder Arsentrioxid nach einem der Ansprüche 6 bis 9 zur Verwendung nach einem der Ansprüche 6 bis 9, wobei es sich bei dem Cu²⁺-Salz um Kupfersulfat oder Kupfer(II)-chlorid handelt.

11. Kombination oder Arsentrioxid nach einem der Ansprüche 6 bis 10 zur Verwendung nach einem der Ansprüche 6 bis 10, wobei die Erkrankung ausgewählt ist aus der Gruppe bestehend aus akuter Promyelozytenleukämie, systemischem Lupus erythematodes (SLE), Multipler Sklerose (MS), Sjögren-Syndrom, rheumatoider Arthritis, Morbus Crohn, Transplantatgegen-Wirt-Erkrankung (GvHD), Myelozytenleukämie, chronischer myeloischer Leukämie, chronischer Lymphozytenleukämie, malignem Gliom, myelodysplastischem Syndrom, multiplem Myelom, und Leber-, Brust-, Bauchspeicheldrüsen- und Prostatakrebs.

12. Zusammensetzung, die im Wesentlichen aus einem Cu²⁺-Salz besteht, zur Verwendung bei der Behandlung einer Erkrankung, ausgewählt aus der Gruppe bestehend aus einer neoplastischen Erkrankung, einer Autoimmunerkrankung, einer entzündlichen Erkrankung und verschiedenen Formen von Multipler Sklerose (MS), bei einem Patienten, der eine Arsenverbindung erhält, derart, dass die Menge des Elements As in einer Tagesdosis zwischen 0,1 µmol/kg und 1,6 µmol/kg beträgt, wobei eine Tagesdosis der Zusammensetzung zwischen 0,05 µmol/kg und 2 µmol/kg an Cu²⁺ umfasst, und wobei das Cu²⁺-Salz die Wirkungen der Arsenverbindung potenziert.

13. Kombination zur Verwendung nach Anspruch 6 oder Zusammensetzung zur Verwendung nach Anspruch 12, wobei die Arsenverbindung ausgewählt ist aus der Gruppe bestehend aus As₂O₃, AsI₃, As₂O₅, As₄O₆, As₂S₂, As₂S₃, As₂S₅, As₄S₄ und Mischungen davon, vorzugsweise Arsentrioxid oder Arsentriiodid.

14. Zusammensetzung nach Anspruch 12 zur Verwendung nach Anspruch 12, wobei die Erkrankung ausgewählt ist aus der Gruppe bestehend aus akuter Promyelozytenleukämie, systemischem Lupus erythematodes (SLE), verschiedenen Formen von Multipler Sklerose (MS), Sjögren-Syndrom, rheumatoider Arthritis, Morbus Crohn, chronischer Transplantat-gegen-Wirt-Erkrankung (GvHD), Myelozytenleukämie, chronischer myeloischer Leukämie, chronischer Lymphozytenleukämie, malignem Gliom, myelodysplastischem Syndrom, multiplem Myelom, und Leber-, Brust-, und Prostatakrebs.

## Revendications

1. Composition comprenant du trioxyde d'arsenic et des ions Cu²⁺, pour son utilisation dans le traitement d'une maladie sélectionnée dans le groupe consistant en une maladie néoplasique, une maladie auto-immune, une maladie inflammatoire et différentes formes de sclérose en plaques (SEP), dans laquelle la quantité de trioxyde d'arsenic dans une dose quotidienne est comprise entre 0,01 et 0,10 mg/kg/jour et dans laquelle la quantité de Cu²⁺ dans une dose quotidienne est comprise entre 0,05 µmol/kg et 2 µmol/kg de Cu²⁺.

2. Composition pour son utilisation selon la revendication 1, dans laquelle les ions Cu²⁺ sont sous la forme d'un sel sélectionné dans le groupe consistant en le sulfate de cuivre et le chlorure de cuivre (II).

3. Composition pour son utilisation selon la revendication 1 ou la revendication 2, dans laquelle une dose quotidienne comprend de 0,01 à 0,05 mg/kg/jour de trioxyde d'arsenic.

4. Composition pour son utilisation selon l'une quelconque des revendications 1 à 3, dans laquelle une dose quotidienne comprend de 0,06 µmol/kg à 2 µmol/kg de Cu²⁺, de préférence de 0,3 µmol/kg à 1,1 µmol/kg de Cu²⁺.

5. Composition pour son utilisation selon l'une quelconque des revendications 1 à 4, dans laquelle la maladie est sélectionnée dans le groupe consistant en une leucémie promyélocytaire aiguë, un lupus érythémateux disséminé (LED), différentes formes de sclérose en plaques (SEP), un syndrome de Sjögren, une polyarthrite rhumatoïde, une maladie de Crohn, une maladie du greffon contre l'hôte (GvHD), une leucémie myélocytaire, une leucémie myéloïde chronique, une leucémie lymphocytaire chronique, un gliome malin, un syndrome myélodysplasique, un myélome chronique, et des cancers du foie, du sein, du pancréas et de la prostate.

6. Combinaison d'un sel de Cu²⁺ et d'un composé d'arsenic, pour son utilisation dans le traitement d'une maladie sélectionnée dans le groupe consistant en une maladie néoplasique, une maladie auto-immune, une maladie inflammatoire et différentes formes de sclérose en plaques (SEP), dans laquelle ledit composé d'arsenic et ledit sel de Cu²⁺ sont administrés à un patient séquentiellement, et dans laquelle la quantité d'As élémentaire dans une dose quotidienne est comprise entre 0,1 µmol/kg et 1,6 µmol/kg et la quantité de Cu²⁺ dans une dose quotidienne est comprise entre 0,05 µmol/kg et 2 µmol/kg.

7. Combinaison d'un sel de Cu²⁺ et de trioxyde d'arsenic, pour son utilisation dans le traitement d'une maladie sélectionnée dans le groupe consistant en une maladie néoplasique, une maladie auto-immune, une maladie inflammatoire et différentes formes de sclérose en plaques (SEP), dans laquelle ledit trioxyde d'arsenic et ledit sel de Cu²⁺ sont administrés à un patient simultanément ou séquentiellement, et dans laquelle la quantité de trioxyde d'arsenic dans une dose quotidienne est comprise entre 0,01 et 0,10 mg/kg/jour et la quantité de Cu²⁺ dans une dose quotidienne est comprise entre 0,05 µmol/kg et 2 µmol/kg.

8. Trioxyde d'arsenic, pour son utilisation dans le traitement d'une maladie sélectionnée dans le groupe consistant en une maladie néoplasique, une maladie auto-immune, une maladie inflammatoire et différentes formes de sclérose en plaques (SEP), dans lequel ledit trioxyde d'arsenic est administré à un patient en combinaison avec un sel de Cu²⁺, en une dose quotidienne telle que la quantité de trioxyde d'arsenic dans une dose quotidienne est comprise entre 0,01 et 0,10 mg/kg/jour et la quantité de Cu²⁺ dans une dose quotidienne est comprise entre 0,05 µmol/kg et 2 µmol/kg.

9. Combinaison selon la revendication 7 ou trioxyde d'arsenic selon la revendication 8, pour leur utilisation selon la revendication 7 ou la revendication 8, dans lesquels la quantité de trioxyde d'arsenic dans une dose quotidienne est comprise entre 0,01 et 0,05 mg/kg/jour.

10. Combinaison du trioxyde d'arsenic selon l'une quelconque des revendications 6 à 9, pour son utilisation selon l'une quelconque des revendications 6 à 9, dans laquelle le sel de Cu²⁺ est un sulfate de cuivre ou un chlorure de cuivre (II).

11. Combinaison du trioxyde d'arsenic selon l'une quelconque des revendications 6 à 10, pour son utilisation selon l'une quelconque des revendications 6 à 10, dans laquelle la maladie est sélectionnée dans le groupe consistant en une leucémie promyélocytaire aiguë, un lupus érythémateux disséminé (LED), une sclérose en plaques (SEP), un syndrome de Sjögren, une polyarthrite rhumatoïde, une maladie de Crohn, une maladie du greffon contre l'hôte (GvHD), une leucémie myélocytaire, une leucémie myéloïde chronique, une leucémie lymphocytaire chronique, un gliome malin, un syndrome myélodysplasique, un myélome multiple et des cancers du foie, du sein, du pancréas et de la prostate.

12. Composition consistant essentiellement en un sel de Cu²⁺, pour son utilisation dans le traitement d'une maladie sélectionnée dans le groupe consistant en une maladie néoplasique, une maladie auto-immune, une maladie inflammatoire et différentes formes de sclérose en plaques (SEP), chez un patient recevant un composé d'arsenic de telle sorte que la quantité d'As élémentaire dans une dose quotidienne est comprise entre 0,1 µmol/kg et 1,6 µmol/kg, dans laquelle une dose quotidienne de la composition comprend de 0,05 µmol/kg à 2 µmol/kg de Cu²⁺ et dans laquelle ledit sel de Cu²⁺ potentialise les effets du composé d'arsenic.

13. Combinaison pour son utilisation selon la revendication 6 ou composition pour son utilisation selon la revendication 12, dans lesquelles le composé d'arsenic est sélectionné dans le groupe consistant en As₂O₃, AsI₃, As₂O₅, As₄O₆, As₂S₂, As₂S₃, As₂S₅, As₄S₄ et les mélanges de ceux-ci, de préférence le trioxyde d'arsenic ou le triiodure d'arsenic.

14. Composition selon la revendication 12, pour son utilisation selon la revendication 12, dans laquelle la maladie est sélectionnée dans le groupe consistant en une leucémie promyélocytaire aiguë, un lupus érythémateux disséminé (LED), différentes formes de sclérose en plaques (SEP), un syndrome de Sjögren, une polyarthrite rhumatoïde, une maladie de Crohn, une maladie du greffon contre l'hôte (GvHD), une leucémie myélocytaire, une leucémie myéloïde chronique, une leucémie lymphocytaire chronique, un gliome malin, un syndrome myélodysplasique, un myélome multiple, et des cancers du foie, du sein et de la prostate.
